# EUROPÄISCHE PATENTANMELDUNG

(11) **EP 4 310 224 A1**
(43) Veröffentlichungstag der Anmeldung: **24.01.2024**
(21) Anmeldenummer: 22185672.7
(22) Anmeldetag: 19.07.2022
(51) Int. Cl.: C25B 1/04, C25B 1/23, C25B 15/08, C01B 3/34, C01B 3/50, C01B 21/38, C01B 32/40, C01B 32/80, C01C 1/04, C07C 201/08, C07C 209/32, C07C 209/36, C07C 263/10, C10K 3/02

(54) **NACHHALTIGE HERSTELLUNG ORGANISCHER AMINOVERBINDUNGEN FÜR DIE PRODUKTION ORGANISCHER ISOCYANATE**

(71) Anmelder: Covestro Deutschland AG, 51373 Leverkusen (DE)
(72) Erfinder: Bulan, Andreas, 51373 Leverkusen (DE); Zabalza, Gaston, 51373 Leverkusen (DE); Weber, Rainer, 51373 Leverkusen (DE); Schmidt, Timm, 51373 Leverkusen (DE); Letmathe, Bernd, 51373 Leverkusen (DE)
(74) Vertreter: Levpat

(57) **Zusammenfassung**

Die Erfindung betrifft ein Verfahren gemäß Anspruch 1, worin Salpetersäure als unmittelbares Verfahrensprodukt eines Verfahrens zur Salpetersäureherstellung, das mindestens folgende Schritte umfasst:
a) Bereitstellung von Wasserstoffgas (5d) und Sauerstoffgas (5b) durch Ausführung einer Wasserelektrolyse (5) in einer Elektrolysevorrichtung unter Einsatz von aus regenerativer Energie hergestellter elektrischer Energie (10) und Wasser (5c);
b) Bereitstellung von Ammoniak (21b) als Verfahrensprodukt einer Umsetzung von zumindest Stickstoff (21a) und Wasserstoffgas (5d) aus besagter Wasserelektrolyse (5) in einer Ammoniaksynthese (21);
c) Herstellung von Salpetersäure (22) durch Umsetzung von zuvor bereitgestelltem Ammoniak (21b) in einer Salpetersäure-Herstellung (22) zumindest mit Sauerstoffhaltigem Gas (5e),
zur Herstellung 22 organischer Aminoverbindungen 17 für die Synthese organischer Isocyanatverbindungen zum Einsatz kommt. Weiter wird die Einbindung dieses Verfahrens in ein Verfahren zur Herstellung organischer Isocyanatverbindungen mit diesen organischen Aminoverbindungen, sowie eine Vorrichtung zur Durchführung des entsprechenden Verfahrens beschrieben.

## Beschreibung

Die Erfindung betrifft ein Verfahren zur nachhaltigen Herstellung organischer Aminoverbindungen für die Synthese organischer Isocyanatverbindungen, worin organische Aminoverbindungen unter Einsatz von grünem Wasserstoff und grünem Ammoniak hergestellt werden, sowie ein Verfahren zur Herstellung organischer Isocyanatverbindungen mit diesen organischen Aminoverbindungen und eine Vorrichtung zur Durchführung des entsprechenden Verfahrens.

Isocyanate werden üblicherweise durch Umsetzung von Aminen mit Phosgen hergestellt, wobei als Nebenprodukt Chlorwasserstoff entsteht. Das für die Phosgensynthese benötigte Kohlenmonoxid wird dabei aus einem Reformierungsprozess unter Einsatz von Erdgas gewonnen, wobei das Chlor für die Phosgensynthese durch das Recycling von Chlorwasserstoff aus der Isocyanat-Herstellung oder durch die Chlor-Alkali-Elektrolyse erzeugt wird.

Die Herstellung der Amine erfolgt über die Hydrierung von Nitroverbindungen, die wiederrum aus Salpetersäure aus den entsprechenden Vorprodukten hergestellt wird. Die Salpetersäure wird dabei aus Ammoniak erzeugt, für dessen Synthese Wasserstoff üblicherweise aus einem Reformerprozess bereitgestellt wird.

Aus Phosgen und Aminen werden die Isocyanate und diese durch Umsetzung mit Polyolen und / oder Polyestern zu Polyurethan-Material umgesetzt.

Polyurethane, nachfolgend auch abgekürzt PU genannt, sind Kunststoffe, die aus der Polyadditionsreaktion von mindestens zwei Hydroxylgruppen enthaltenden Polyolen mit Polyisocyanaten entstehen. Polyisocyanate werden nachfolgend, wenn nicht anders aufgeführt, als Isocyanate bezeichnet. Amine zur Herstellung der Isocyanate sind ebenfalls Polyamine und werden als Amine bezeichnet. Der Einsatz von Diolen und Diisocyanaten führt zu linearen Polyurethanen. Vernetzte Polyurethane können durch Umsetzung von Triisocyanat-Diisocyanat-Gemischen mit Triol-Diol-Gemischen hergestellt werden. Die Eigenschaften von PU können in einem weiten Rahmen variiert werden. Je nach Vernetzungsgrad und/oder eingesetzter Isocyanat- oder OH-Komponente erhält man Duroplaste, Thermoplaste oder Elastomere. Polyurethane werden jedoch auch als Formmassen zum Formpressen, als Gießharze (Isocyanat-Harze), als (textile) elastische Faserstoffe, Polyurethanlacke und als Polyurethanklebstoffe verwendet. Aus Polyurethan lassen sich auch sehr einfach Schaumstoffe herstellen.

Weiche PU-Schaumstoffe werden für sehr viele Zwecke verwendet, vor allem als Polstermaterial z. B. für Möbel und Autositze, als Matratzenschaum, als Teppichrückenmaterial, zur Textilkaschierung, als Reinigungsschwamm oder als Filtermaterial.

PU-Hartschäume werden vor allem zur Wärmedämmung z. B. in Gebäuden, Kühlgeräten, Wärme- und Kältespeichern sowie einigen Rohrsystemen (Kunststoffmantelverbundrohr, flexible Verbundrohre) eingesetzt.

Weitere, relativ neue Anwendungsgebiete für PU-Schäume gibt es im Fahrzeugbau wie Lenkrad, Armauflage, Softbeschichtung von Handgriffen, Innenraumverkleidung, Armaturenbrett, Schalldämmung, Klapperschutz, Abdichtungen, Transparentbeschichtung von Holzdekoren.

Aufgabe der vorliegenden Erfindung war es, ein nachhaltigeres Verfahren zur Synthese organischer Aminoverbindungen für die Isocyanatherstellung und letztlich auch für die Polyurethan-Herstellung zu finden, die ohne großen Mehraufwand in bestehenden Produktionsprozessen verwendet werden kann.

Unter "Nachhaltigkeit" eines Verfahrens versteht der Fachmann in Anwendung der durch die UN geprägte Nachhaltigkeits-Definition ("sustainable development") gemäß Brundtlandbericht der "Weltkommission für Umwelt und Entwicklung", dass durch die Ausführung des Verfahrens in der Gegenwart ein möglichst geringer bis gar kein Beitrag geleistet wird, dass künftige Generationen der Menschheit ihre eigenen Bedürfnisse nicht mehr befriedigen können, insbesondere Bedürfnisse mit Blick auf die Nutzung von Ressourcen wie z.B. fossiler Rohstoffe und insbesondere mit Blick auf die Schonung des Lebensraumes, wie z.B. den Schutz der Erdatmosphäre. Die Erfindung hat somit zur Aufgabe, die Produktion von organischen Aminen, sowie daraus hergestelltem, organischem Isocyanat und Polyurethan nachhaltiger als die aus dem Stand der Technik bekannten Produktionsmethoden zu gestalten. Der Beitrag der Produktion von Isocyanat und Polyurethan zu einer sinkenden Befriedigung der Bedürfnisse zukünftiger Generationen soll vermindert bis vermieden werden.

Ein Gegenstand der vorliegenden Erfindung ist ein Verfahren zur Herstellung von organischer Aminoverbindung für die Synthese organischer Isocyanatverbindungen, enthaltend mindestens die Schritte
i) Bereitstellung mindestens einer organischen Nitroverbindung als Verfahrensprodukt eines Verfahrens zur Synthese mindestens einer organischen Nitroverbindung, das mindestens folgende Schritte umfasst:
   i-1) Bereitstellung von Salpetersäure als unmittelbares Verfahrensprodukt eines Verfahrens zur Salpetersäureherstellung, das mindestens folgende Schritte umfasst:
      a) Bereitstellung von Wasserstoffgas und Sauerstoffgas durch Ausführung einer Wasserelektrolyse in einer Elektrolysevorrichtung unter Einsatz von aus regenerativer Energie hergestellter elektrischer Energie und Wasser;
      b) Bereitstellung von Ammoniak als unmittelbares Verfahrensprodukt einer Umsetzung von zumindest Stickstoff und Wasserstoffgas aus besagter Wasserelektrolyse in einer Ammoniaksynthese;
      c) Herstellung von Salpetersäure durch Umsetzung von zuvor hergestelltem Ammoniak in einer Salpetersäure-Herstellung zumindest mit Sauerstoffhaltigem Gas;
   i-2) Synthese mindestens einer organischen Nitroverbindung, bevorzugt mindestens einer aromatischen organischen Nitroverbindung, zumindest durch Nitrierung mindestens einer organischen Verbindung, bevorzugt mindestens einer aromatischen organischen Verbindung, mit mindestens der besagten bereitgestellten Salpetersäure;
ii) Bereitstellung von Wasserstoffgas, bevorzugt durch Ausführung einer Wasserelektrolyse in einer Elektrolysevorrichtung unter Einsatz von aus regenerativer Energie hergestellter elektrischer Energie;
iii) Einbringung der mindestens einen bereitgestellten organischen Nitroverbindung und des bereitgestellten Wasserstoffgases in eine Hydrierung und anschließende Hydrierung durch Reduktion von Nitrogruppen der besagten mindestens einen organischen Nitroverbindung über deren Umsetzung mit besagtem Wasserstoffgas unter Erhalt mindestens einer organischen Aminoverbindung, bevorzugt mindestens einer aromatischen organischen Aminoverbindung.

Für die Bereitstellung der mindestens einen organischen Nitroverbindung gemäß Schritt i) ist es ausreichend, wenn die mindestens eine organische Nitroverbindung zwingend ein unmittelbares Verfahrensprodukt mindestens der Schritte i-1) und i-2) des vorgenannten Verfahrens ist. Dies bedeutet, dass es für die Ausführung des Schrittes der Bereitstellung der mindestens einen organischen Nitroverbindung bereits ausreicht, die besagte organische Nitroverbindung als ein unmittelbares Verfahrensprodukt mindestens der Schritte i-1) und i-2) lediglich im Rahmen einer Belieferung als Rohstoff aus einem Lagerbehälter oder aus einer Zuleitung zu entnehmen. In diesem Falle führt der Hersteller des organischen Amins nach dem erfindungsgemäßen Verfahren die vorgenannten Schritte zur Herstellung der mindestens einen organischen Nitroverbindung nicht selbst aus, sondern er sorgt dafür, dass eine zur Herstellung des organischen Amins bereitgestellte organische Nitroverbindung durch Anwendung eines Verfahrens mit mindestens den Schritten i-1) und i-2) hergestellt wurde und somit dessen unmittelbares Verfahrensprodukt ist.

Im Falle der Belieferung wird bevorzugt das Verfahren derart durchgeführt, dass in Schritt i) die organische Nitroverbindung als Verfahrensprodukt der besagten Synthese des Schritts i-2) in einem beweglichen, abgeschlossenem Transportbehälter bereitgestellt wird, gegebenenfalls daraus zur Zwischenlagerung in Lagerbehälter überführt wird, wobei die besagte Synthese und die Hydrierung gemäß Schritt iii) nicht miteinander in Fluidverbindung stehen.

Unter einer Fluidverbindung wird ein Teil einer Vorrichtung verstanden, der andere Vorrichtungsteile miteinander verbindet und durch den sich ein Stoff, der in jedem Aggregatzustand vorliegen kann, als Stoffstrom von einem Vorrichtungsteil zum nächsten transportieren lässt, beispielsweise eine Zuleitung in Form eines Rohres. Der Begriff "in Fluidverbindung stehen" bedeutet, dass benannte Vorrichtungsteile über eine Fluidverbindung miteinander verbunden sind.

Besagter beweglicher, abgeschlossener Transportbehälter kann beispielsweise ein Kessel eines Kesselwaggons, eines Tankschiffs oder eines Tanklastkraftwagens sein. Unter abgeschlossen wird verstanden, dass der besagte Transportbehälter an dessen Vorrichtung zur Befüllung bzw. Entnahme des Transportgutes während des Transports verschlossen ist.

Als Lagerbehälter gilt erfindungsgemäß ein Behälter, der an einen festen Ort gebunden ist, wie beispielsweise ein Tanklager. Lagerbehälter gehören zum unbeweglichen Anlagevermögen oder sind ein Teil davon.

Im Rahmen einer erfindungsgemäß bevorzugten Ausführungsform des erfindungsgemäßen Verfahrens wird die organische Nitroverbindung als unmittelbares Verfahrensprodukt von besagter Synthese des Schritts i-2) in Form eines Stoffstromes direkt über eine Fluidverbindung aus der besagten Synthese über eine gegebenenfalls notwendige Zwischenlagerung in mindestens einem Lagerbehälter bereitgestellt, wobei die besagte Synthese, der gegebenenfalls notwendige mindestens eine Lagerbehälter und die Hydrierung gemäß Schritt iii) miteinander in Fluidverbindung stehen. Ein Verfahren dieser bevorzugten Ausführungsform enthält als besonders bevorzugte Ausführungsform mindestens die Schritte
i) Herstellung mindestens einer organischen Nitroverbindung durch mindestens folgende Schritte:
   i-1) Bereitstellung von Salpetersäure als unmittelbares Verfahrensprodukt eines Verfahrens zur Salpetersäureherstellung, das mindestens folgende Schritte umfasst:
      a) Bereitstellung von Wasserstoffgas und Sauerstoffgas durch Ausführung einer Wasserelektrolyse in einer Elektrolysevorrichtung unter Einsatz von aus regenerativer Energie hergestellter elektrischer Energie und Wasser;
      b) Bereitstellung von Ammoniak als unmittelbares Verfahrensprodukt einer Umsetzung von zumindest Stickstoff und Wasserstoffgas aus besagter Wasserelektrolyse in einer Ammoniaksynthese;
      c) Herstellung von Salpetersäure durch Umsetzung von zuvor bereitgestelltem Ammoniak in einer Salpetersäure-Herstellung zumindest mit Sauerstoff-haltigem Gas;
   i-2) Synthese mindestens einer organischen Nitroverbindung, bevorzugt mindestens einer aromatischen organischen Nitroverbindung, zumindest durch Nitrierung mindestens einer organischen Verbindung, bevorzugt mindestens einer aromatischen organischen Verbindung, mit mindestens der besagten bereitgestellten Salpetersäure;
ii) Bereitstellung von Wasserstoffgas, bevorzugt durch Ausführung einer Wasserelektrolyse in einer Elektrolysevorrichtung unter Einsatz von aus regenerativer Energie hergestellter elektrischer Energie;
iii) Einbringung der organischen Nitroverbindung und des bereitgestellten Wasserstoffgases in eine Hydrierung und anschließende Hydrierung durch Reduktion von Nitrogruppen der besagten mindestens einen organischen Nitroverbindung über deren Umsetzung mit besagtem Wasserstoffgas unter Erhalt mindestens einer organischen Aminoverbindung, bevorzugt mindestens einer aromatischen organischen Aminoverbindung, mit der Maßgabe, dass die organische Nitroverbindung in Form eines Stoffstromes über eine Fluidverbindung aus der besagten Synthese des Schrittes i-2), gegebenenfalls über eine Zwischenlagerung in mindestens einem Lagerbehälter, eingebracht wird, wobei die besagte Synthese und die Hydrierung gemäß Schritt iii) sowie gegebenenfalls der mindestens eine Lagerbehälter, miteinander in Fluidverbindung stehen.

Die Synthese der mindestens einen organischen Nitroverbindung erfolgt gemäß Schritt i-2) ausgehend von besagter organischen Verbindung und besagter Salpetersäure in einer dem Fachmann notorisch bekannten Weise. Beispielsweise können durch die Synthese in Schritt i-2) organische Mononitroverbindungen, insbesondere organische, aromatische Mononitroverbindungen, erhalten werden, wie in EP 0 668 263 A1 beschrieben, auf die hier ausdrücklich und vollinhaltlich Bezug genommen wird. Als bevorzugte organische Nitroverbindungen ist Mononitrobenzol zu nennen. Weiterhin können mittels der Synthese des Schrittes i-2) organische Dinitroverbindungen bereitgestellt werden, wie beispielsweise Dinitrotoluol (DNT). Die Herstellung von Dinitrotoluol ist dem Fachmann notorisch bekannt, wie beispielsweise aus den Druckschriften Ullmann's Enzyklopädie der technischen Chemie, 4.Auflage, Band 17, Seite 391 ff, 1979, Verlag Chemie Weinheim / New York; H.Hermann, J.Gebauer, P. Konieczny, "Industrial Nitration of Toluene to Dinitrotoluene" in ACS-Symposium, Series 623, 234-249, 1996, ed. L.F.Albright, R.V.C oder aus EP 1 880 989 A1, auf die hier ausdrücklich und vollinhaltlich Bezug genommen wird.

Zur Bereitstellung der organischen Nitroverbindung wird gemäß Schritt i-2) eine organische Verbindung, bevorzugt mindestens eine aromatische organische Verbindung, unter Einsatz mindestens der besagten bereitgestellten Salpetersäure nitriert. Die dabei eingesetzte organische Verbindung wird besonders bevorzugt ausgewählt aus Benzol oder Toluol.

Im Rahmen einer weiteren Ausführungsform hat es sich als verbessert nachhaltig erwiesen, wenn mindestens eine in Schritt i-2) eingesetzte organische Verbindung eine aromatische, organische Verbindung ist, die als unmittelbares Verfahrensprodukt eines Verfahrens bereitgestellt wird, umfassend mindestens folgende Schritte:
Pyrolyse von organischem Material, bei einer Temperatur von 600 bis 1000°C unter Erhalt von gasförmigem Pyrolysat und Pyrolyserückstand;
Kontaktieren des gasförmigen Pyrolysates mit einem heterogen Katalysator unter Erhalt von Konversionsprodukt, enthaltend mindestens eine aromatische, organische Verbindung mit einem Molekulargewicht von weniger als 300 g/mol;
Aufarbeitung des Konversionsproduktes unter Erhalt der besagten aromatischen, organischen Verbindung, insbesondere ausgewählt aus Benzol, Toluol oder Xylol.

Ein entsprechendes Pyrolyseverfahren zur Bereitstellung wird beispielsweise in der Druckschrift WO 2020/204707 A1 beschrieben, auf die in dieser Anmeldung ausdrücklich und vollinhaltlich Bezug genommen wird. Hierin sind ebenso für den Prozess geeignete heterogene Katalysatoren zur Herstellung des Konversionsproduktes beschrieben.

Als organisches Material kann in der Pyrolyse beispielsweise Kunststoff, wie Polyurethan-Material und/oder Polycarbonat-Material, eingesetzt werden. Dieser Kunststoff ist bevorzugt Kunststoff-Abfall, wie beispielsweise Polyurethan-Material Abfall". Der "Kunststoff-Abfall", insbesondere "Polyurethan-Material Abfall", kann durch Nutzung von Kunststoff, insbesondere Polyurethan, im Markt entstanden sein. Im Falle von Polyurethan-Material Abfall wurde wiederum bevorzugt das Polyurethan aus Diisocyanaten hergestellt, welche aus nach dem erfindungsgemäßen Verfahren bereitgestellten organischen Aminen hergestellt wurden. Bei Wiederverwertung eines solchen Polyurethan-Material Abfalles in dem erfindungsgemäßen Verfahren spricht man von einem sogenannten "closed loop" Verfahren. Allerdings ist es selbstverständlich auch möglich in dieser Ausführungsform des erfindungsgemäßen Verfahrens solchen Polyurethan-Material Abfall zu verwerten, der aus Polyurethanen stammt, die nicht aus Diisocyanaten aus dem erfindungsgemäßen Verfahren hergestellt wurden.

Weiter verbessert ist ein entsprechendes Pyrolyseverfahren zur Bereitstellung aromatischer organischer Verbindung, in dem der bei der Pyrolyse anfallende Pyrolyserückstand in einer Gasifizierung unter partieller Oxidation zu Kohlenmonoxid, Wasserstoff und Reststoff umgewandelt wird und das gebildete Kohlenmonoxid nach optionaler Aufarbeitung einer Phosgensynthese und der Wasserstoff in die Bereitstellung von Wasserstoff gemäß Schritt ii) des Verfahrens eingebracht wird.

Die Synthese der mindestens einen organischen Nitroverbindung erfolgt gemäß Schritt i-2) ausgehend von mindestens einer organischen Verbindung und von Salpetersäure. Gemäß Schritt i-1) des erfindungsgemäßen Verfahrens und dessen weiteren Ausführungsformen wird für diese Synthese des Schrittes i-2) Salpetersäure bereitgestellt. Für die Bereitstellung der mindestens einen organischen Nitroverbindung ist es dabei ausreichend, wenn die bereitgestellte Salpetersäure zwingend ein unmittelbares Verfahrensprodukt mindestens der Schritte i-1) a) bis c) des Verfahrens dieser bevorzugten Ausführungsform bzw. des allgemeinem Verfahrens der Erfindung ist.

Dies bedeutet, dass es für die Ausführung des Schrittes der Bereitstellung der Salpetersäure bereits ausreicht, die besagte Salpetersäure als ein unmittelbares Verfahrensprodukt eines Verfahrens mit mindestens den Schritten i-1) a) bis i-1) c) lediglich im Rahmen einer Belieferung als Rohstoff aus einem Lagerbehälter oder aus einer Zuleitung zu entnehmen. In diesem Falle führt der Hersteller des organischen Amins oder der organischen Nitroverbindung nach dem erfindungsgemäßen Verfahren die vorgenannten Schritte zur Herstellung der Salpetersäure nicht selbst aus, sondern er sorgt nur dafür, dass die zur Herstellung des organischen Amins bzw. zur Herstellung der organischen Nitroverbindung bereitgestellte Salpetersäure durch Anwendung mindestens durch die Schritte i-1) a) bis c) hergestellt wurde und somit deren unmittelbares Verfahrensprodukt ist. Im Falle der Belieferung wird bevorzugt das Verfahren derart durchgeführt, dass in Schritt i-1) die Salpetersäure als unmittelbares Verfahrensprodukt der besagten Salpetersäureherstellung in einem beweglichen, abgeschlossenem Transportbehälter bereitgestellt wird, gegebenenfalls daraus zur Zwischenlagerung in Lagerbehälter überführt wird, wobei die besagte Salpetersäureherstellung und die Synthese gemäß Schritt ii) nicht miteinander in Fluidverbindung stehen.

Ebenso ist es erfindungsgemäß möglich, wenn zur Bereitstellung der Salpetersäure mindestens eine der vorgenannten Reaktionsschritte zur Herstellung der Salpetersäure als integrale Schritte eines erfindungsgemäßen Verfahrens zur Herstellung von organischem Amin ausgeführt werden und die dabei erhaltene Salpetersäure, gegebenenfalls nach einer Zwischenlagerung in einem Lagerbehälter, der Synthese der organischen Nitroverbindung zugeführt wird. Dabei ist es wiederum bevorzugt, wenn in Schritt i-1) die Salpetersäure als Verfahrensprodukt besagter Salpetersäureherstellung in Form eines Stoffstromes direkt über eine Fluidverbindung aus der besagten Salpetersäureherstellung über eine gegebenenfalls notwendige Zwischenlagerung in mindestens einem Lagerbehälter bereitgestellt wird, wobei die besagte Salpetersäureherstellung, der gegebenenfalls notwendige mindestens eine Lagerbehälter und die Synthese gemäß Schritt i-2) über eine Fluidverbindung miteinander verbunden sind.

Ein Verfahren dieser bevorzugten Ausführungsform enthält als besonders bevorzugte Ausführungsform mindestens die Schritte
i) Herstellung mindestens einer organischen Nitroverbindung durch mindestens folgende Schritte:
   i-1) Herstellung von Salpetersäure als unmittelbares Verfahrensprodukt eines Verfahrens zur Salpetersäureherstellung, das mindestens folgende Schritte umfasst:
      a) Bereitstellung von Wasserstoffgas und Sauerstoffgas durch Ausführung einer Wasserelektrolyse in einer Elektrolysevorrichtung unter Einsatz von aus regenerativer Energie hergestellter elektrischer Energie und Wasser;
      b) Bereitstellung von Ammoniak als unmittelbares Verfahrensprodukt einer Umsetzung von zumindest Stickstoff und Wasserstoffgas aus besagter Wasserelektrolyse in einer Ammoniaksynthese;
      c) Herstellung von Salpetersäure durch Umsetzung von zuvor bereitgestelltem Ammoniak in einer Salpetersäure-Herstellung zumindest mit Sauerstoff-haltigem Gas;
   i-2) Synthese mindestens einer organischen Nitroverbindung, bevorzugt mindestens einer aromatischen organischen Nitroverbindung, zumindest durch Nitrierung mindestens einer organischen Verbindung, bevorzugt mindestens einer aromatischen organischen Verbindung, mit mindestens der besagten bereitgestellten Salpetersäure;
ii) Bereitstellung von Wasserstoffgas, bevorzugt durch Ausführung einer Wasserelektrolyse in einer Elektrolysevorrichtung unter Einsatz von aus regenerativer Energie hergestellter elektrischer Energie;
iii) Einbringung der organischen Nitroverbindung und des bereitgestellten Wasserstoffgases in eine Hydrierung und anschließende Hydrierung durch Reduktion von Nitrogruppen der besagten mindestens einen organischen Nitroverbindung über deren Umsetzung mit besagtem Wasserstoffgas unter Erhalt mindestens einer organischen Aminoverbindung, bevorzugt mindestens einer aromatischen organischen Aminoverbindung, mit der Maßgabe, dass zum einen in Schritt i-1) die Salpetersäure als unmittelbares Verfahrensprodukt besagter Salpetersäureherstellung in Form eines Stoffstromes direkt über eine Fluidverbindung aus der besagten Salpetersäureherstellung über eine gegebenenfalls notwendige Zwischenlagerung in mindestens einem Lagerbehälter bereitgestellt wird, wobei die besagte Salpetersäureherstellung, der gegebenenfalls notwendige mindestens eine Lagerbehälter und die Synthese gemäß Schritt ii) über eine Fluidverbindung miteinander verbunden sind und zum anderen die organische Nitroverbindung in Form eines Stoffstromes über eine Fluidverbindung aus der besagten Synthese des Schrittes i-2), gegebenenfalls über eine Zwischenlagerung in mindestens einem Lagerbehälter, eingebracht wird, wobei die besagte Synthese und die Hydrierung gemäß Schritt iii) sowie gegebenenfalls der mindestens eine Lagerbehälter, miteinander in Fluidverbindung stehen.

Die Herstellung der Salpetersäure erfolgt gemäß Schritt i-1) c) ausgehend von besagtem Ammoniak und besagtem sauerstoffhaltigem Gas (beispielsweise Luft oder Sauerstoffgas) in einer dem Fachmann notorisch bekannten Weise, beispielsweise wie in der Druckschrift EP 0 808 797 A2 beschrieben, auf die hier ausdrücklich und vollinhaltlich Bezug genommen wird. Bevorzugt wird zur Herstellung der Salpetersäure in Schritt i-1) c) Sauerstoffgas als sauerstoffhaltiges Gas eingesetzt.

Gemäß Schritt i-1) b) des erfindungsgemäßen Verfahrens und dessen weiteren Ausführungsformen wird Ammoniak für den Schritt i-1) c) bereitgestellt. Für die Bereitstellung der mindestens einer organischen Nitroverbindung gemäß Schritt i-1) ist es dabei ausreichend, wenn der für die Bereitstellung von Salpetersäure bereitgestellte Ammoniak zwingend ein unmittelbares Verfahrensprodukt mindestens des Schrittes i-1) b) ist. Dies bedeutet, dass es für die Ausführung des Schrittes der Bereitstellung des Ammoniaks bereits ausreicht, den besagten Ammoniak als ein unmittelbares Verfahrensprodukt mindestens des Schrittes i-1) b) lediglich im Rahmen einer Belieferung als Rohstoff aus einem Lagerbehälter oder aus einer Zuleitung zu entnehmen. In diesem Falle führt der Hersteller des organischen Amins oder der organischen Nitroverbindung nach dem erfindungsgemäßen Verfahren die vorgenannten Schritte zur Herstellung des Ammoniaks nicht selbst aus, sondern er sorgt nur dafür, dass der zur Herstellung des organischen Amins bzw. zur Herstellung der organischen Nitroverbindung bereitgestellte Ammoniak durch Anwendung eines Verfahrens mindestens mit Schritt i-1) b) hergestellt wurde und somit dessen unmittelbares Verfahrensprodukt ist.

Es ist erfindungsgemäß möglich, wenn zur Bereitstellung des Ammoniaks mindestens eine der vorgenannten Reaktionsschritte zur Herstellung des Ammoniaks als integrale Schritte eines erfindungsgemäßen Verfahrens zur Herstellung von organischem Amin ausgeführt werden und der dabei erhaltene Ammoniak, gegebenenfalls nach einer Zwischenlagerung in einem Lagerbehälter, der Bereitstellung bzw. Herstellung der Salpetersäure zugeführt wird. Dabei ist es wiederum bevorzugt, wenn in Schritt i-1) b) der Ammoniak als unmittelbares Verfahrensprodukt besagter Ammoniakherstellung in Form eines Stoffstromes direkt über eine Fluidverbindung aus der besagten Herstellung des Ammoniaks über eine gegebenenfalls notwendige Zwischenlagerung in mindestens einem Lagerbehälter bereitgestellt wird, wobei die besagte Herstellung des Ammoniaks, der gegebenenfalls notwendige mindestens eine Lagerbehälter und die Synthese gemäß Schritt ii) über eine Fluidverbindung miteinander verbunden sind.

In denjenigen Ausführungsformen der Erfindung, in denen die Salpetersäure direkt als Stoffstrom aus der Salpetersäureherstellung über eine Fluidverbindung zwischen der Synthese des Schrittes i-2) und der Hydrierung gemäß Schritt iii) sowie gegebenenfalls dem mindestens eine Lagerbehälter bereitgestellt wird, hat es sich zusätzlich als bevorzugt herausgestellt, wenn in Schritt i-1) der Ammoniak als unmittelbares Verfahrensprodukt der besagten Herstellung von Ammoniak in einem beweglichen, abgeschlossenem Transportbehälter bereitgestellt wird, gegebenenfalls daraus zur Zwischenlagerung in Lagerbehälter überführt wird, wobei die besagte Herstellung von Ammoniak und die Salpetersäureherstellung nicht miteinander in Fluidverbindung stehen. Hierbei ist es wiederum besonders bevorzugt, wenn zur Herstellung von organischen Aminen für die Synthese organischer Isocyanatverbindungen, mindestens die Schritte ausgeführt werden:
i) Herstellung mindestens einer organischen Nitroverbindung durch ein Verfahren zur Synthese mindestens einer organischen Nitroverbindung, das mindestens folgende Schritte umfasst:
   i-1) Herstellung von Salpetersäure durch Ausführung eines Verfahrens zur Salpetersäureherstellung, das mindestens folgende Schritte umfasst:
      a) und b) Bereitstellung von Ammoniak als unmittelbares Verfahrensprodukt einer Umsetzung von zumindest Stickstoff und Wasserstoffgas, welches neben Sauerstoffgas durch Ausführung einer Wasserelektrolyse in einer Elektrolysevorrichtung unter Einsatz von aus regenerativer Energie hergestellter elektrischer Energie und Wasser hergestellt wurde;
      c) Herstellung von Salpetersäure in einer Salpetersäure-Herstellung durch Umsetzung von zuvor bereitgestelltem Ammoniak zumindest mit Sauerstoff-haltigem Gas;
   i-2) Synthese mindestens einer organischen Nitroverbindung, bevorzugt mindestens einer aromatischen organischen Nitroverbindung, zumindest durch Nitrierung mindestens einer organischen Verbindung, bevorzugt mindestens einer aromatischen organischen Verbindung, mit mindestens der besagten Salpetersäure;
ii) Bereitstellung von Wasserstoffgas, bevorzugt durch Ausführung einer Wasserelektrolyse in einer Elektrolysevorrichtung unter Einsatz von aus regenerativer Energie hergestellter elektrischer Energie;
iii) Einbringung der mindestens einen organischen Nitroverbindung und des bereitgestellten Wasserstoffgases in eine Hydrierung und anschließende Hydrierung durch Reduktion von Nitrogruppen der besagten mindestens einen organischen Nitroverbindung über deren Umsetzung mit besagtem Wasserstoffgas unter Erhalt mindestens einer organischen Aminoverbindung, bevorzugt mindestens einer aromatischen organischen Aminoverbindung.

Die Herstellung des Ammoniaks erfolgt gemäß Schritt i-1) b) ausgehend von Stickstoff und in Schritt i-1) a) bereitgestelltem Wasserstoff in einer dem Fachmann notorisch bekannten Weise, wie beispielsweise nach dem Haber-Bosch-Verfahren unter Anwendung bekannter Reaktortechnologien und -designs für dieses Verfahren beschrieben, auf die hier ausdrücklich und vollinhaltlich Bezug genommen wird.

Für die Bereitstellung bzw. die Herstellung der Salpetersäure hat es sich als bevorzugt erwiesen, wenn der zur Herstellung von Ammoniak in Schritt i-1) b) genutzte Stickstoff durch eine Luftzerlegung bereitgestellt wird. Prozesse zur Luftzerlegung und entsprechend geeignete Anlagen zur Stickstoffherstellung gehören zum Stand der Technik und sind auf dem Markt erhältlich.

Der in der Oxidationsreaktion der Salpetersäureherstellung genutzte Sauerstoff kann ebenso durch eine Luftzerlegung bereitgestellt werden. Daher ist im Rahmen einer weiteren Ausführungsform ein bevorzugtes Verfahren dadurch gekennzeichnet, dass die in Schritt i-1) bereitgestellte Salpetersäure ein Verfahrensprodukt einer Salpetersäureherstellung ist, in der für die Oxidationsreaktion des Schritts c) mindestens ein Sauerstoff-haltiges Gas (5e), ausgewählt aus Sauerstoff aus einer Wasserelektrolyse, Luft oder Sauerstoff aus einer Luftzerlegung eingesetzt wird. Wenn eine Luftzerlegung zur Sauerstoffbereitstellung für die Oxidation eingesetzt wird, dann ist es besonders bevorzugt, wenn die in Schritt i-1) bereitgestellte Salpetersäure ein unmittelbares Verfahrensprodukt einer Salpetersäureherstellung ist, in der für die Oxidationsreaktion des Schritts c) Sauerstoff für das Sauerstoff-haltig Gas durch eine Luftzerlegung die ebenfalls Stickstoff für Schritt i-1) b) liefert, bereitgestellt wird. Die Bereitstellung von Wasserstoffgas in Schritt i-1) a) des Verfahrens im Rahmen der Herstellung des Ammoniaks für die Salpetersäureherstellung erfolgt zwingend durch eine Wasserelektrolyse, die teilweise oder vollständig mit elektrischem Strom betrieben wird, der unter Einsatz von regenerativer Energie als Energiequelle bereitgestellt wurde.

Die Bereitstellung von Wasserstoffgas in Schritt ii) des Verfahrens für die Hydrierung in Schritt iii) des Verfahrens kann beispielsweise durch Wasserelektrolyse oder durch eine Elektrolyse zur Herstellung von Chlor oder durch eine Kombination davon erfolgen, wobei besagte ausgewählte Elektrolyse jeweils teilweise oder vollständig mit elektrischem Strom betrieben wird, der unter Einsatz von regenerativer Energie als Energiequelle bereitgestellt wurde. Dabei hat es sich im Rahmen einer Ausführungsform des Verfahrens als vorteilhaft erwiesen, wenn der in Schritt ii) bereitgestellte Wasserstoff mindestens durch eine Elektrolyse von Wasser unter Einsatz von elektrischer Energie hergestellt wird, wobei die Bereitstellung elektrischer Energie als regenerative Energie wahlweise Windkraft, Sonnenenergie, Wasserkraft oder Mischungen daraus, eingesetzt wird.

Unter "regenerativer Energie" versteht der Fachmann Energie aus einer Energiequelle, die sich nicht erschöpft, wie z.B. Windenergie, Wasserenergie, Bioenergie (z.B. Verstromung von Biogas oder Biomasse) oder Sonnenenergie. Als regenerative Energie eignen sich daher ganz besonders bevorzugt wahlweise Windkraft, Sonnenenergie, Wasserkraft oder Mischungen daraus. Wird regenerative Energie im Rahmen der vorliegenden Erfindung als Energiequelle, insbesondere für die Bereitstellung von elektrischem Strom, genutzt, dann kann es für die Gewährleistung einer Ausführung des erfindungsgemäßen Verfahrens in einer Mangelperiode von regenerativer Energie vorteilhaft sein, zu einem Zeitpunkt der Verfügbarkeit von regenerativer Energie diese zur Überbrückung von Mangelperioden zu speichern, z.B. in Form von elektrischem Strom in einem Akku.

Die Wasserelektrolyse zur Herstellung von Wasserstoffgas stellt die elektrochemische Umwandlung von Wasser dar. Unter Nutzung von Strom wird hier Wasser in Wasserstoff und Sauerstoff gespalten. Bekannte Verfahren sind die Protonen-Austausch-Elektrolyse (PEM-Elektrolyse), welche bspw. in EP 3489394 A beschrieben wird. Ein weiteres Verfahren stellt die alkalische Wasserelektrolyse dar. Beide Verfahren unterscheiden sich durch insbesondere durch die Verfahrensparameter wie bspw. Temperatur, Druck und pH-Wert. Beide Verfahren führen jedoch zu den Zielprodukten Wasser- und Sauerstoffgas. Die Wasserelektrolyse kann mit Anlagen nach dem Stand der Technik durchgeführt werden. Bekannt und kommerziell erhältlich sind technische Anlagen zur alkalischen Wasserelektrolyse als auch zur PEM-Elektrolyse. Die Prinzipien der Wasserelektrolyse sind beispielhaft in Kapitel 6.3.4 in Volkmar M. Schmidt in "Elektrochemische Verfahrenstechnik" (2003 Wiley-VCH-Verlag; ISBN 3-527-29958-0) beschrieben.

In Schritt iii) des erfindungsgemäßen Verfahrens erfolgt die Einbringung der mindestens einen organischen Nitroverbindung aus Schritt i) und des in Schritt ii) des Verfahrens bereitgestellten Wasserstoffgases in eine Hydrierung. Im Anschluss daran erfolgt die Hydrierung durch Reduktion von Nitrogruppen der besagten mindestens einen organischen Nitroverbindung über deren Umsetzung mit besagtem Wasserstoffgas.

Die Durchführung einer Hydrierung organischer Nitroverbindungen mit Wasserstoffgas ist dem Fachmann notorisch bekannt. Entsprechend für den Schritt iii) heranziehbare und nutzbare Umsetzungen zur Hydrierung kennt der Fachmann beispielsweise aus den Druckschriften Gerald Booth (2007), Ullmanns Enzyklopädie der Industriechemie, Weinheim, Wiley VCH, doi:10.1002 / 14356007.a17_411, auf die hier ausdrücklich und vollinhaltlich Bezug genommen wird.

Eine weitere, erfindungsgemäß bevorzugte Ausführungsform des Verfahrens ist dadurch gekennzeichnet, dass die in Schritt iii) erhaltene organische Aminoverbindung ausgewählt wird aus Toluylendiamin (TDA), Methylendi(phenylamin) (MDA), wiederum bevorzugt ausgewählt aus Diphenylmethan-2,2'-diamin, Diphenylmethan-2,4'-diamin, Diphenylmethan-4,4'-diamin oder Mischungen daraus) oder Mischungen daraus.

Die durch das erfindungsgemäße Verfahren hergestellten organischen Aminoverbindungen eignen sich bevorzugt für die Synthese von organischem Isocyanat. Im Rahmen einer weiteren bevorzugten Ausführungsform des erfindungsgemäßen Verfahrens werden für die Synthese organischen Isocyanats zusätzlich mindestens folgende Schritte ausgeführt:
iv) Synthese von Phosgen aus zumindest Kohlenmonoxid und Chlorgas;
v) Isocyanatherstellung von organischem Isocyanat durch Umsetzung zumindest des Phosgens mit der mindestens einen organischen Aminoverbindung, unter Freisetzung von Chlorwasserstoff.

Ein weiterer Erfindungsgegenstand ist daher ein Verfahren zur Synthese organischen Isocyanats, enthaltend mindestens die Schritte
Bereitstellung mindestens einer organischen Aminoverbindung als unmittelbares Verfahrensprodukt des zuvor beschriebenen Verfahrens zur Herstellung von organischen Aminen für die Synthese organischer Isocyanatverbindungen;
Synthese von Phosgen aus zumindest Kohlenmonoxid und Chlorgas;
Isocyanatherstellung von organischem Isocyanat durch Umsetzung zumindest des Phosgens mit der mindestens einen organischen Aminoverbindung, unter Freisetzung von Chlorwasserstoff.

Im Rahmen der Bereitstellung mindestens einer organischen Aminoverbindung wird ein Verfahrensprodukt des zuvor beschriebenen Verfahrens zur Herstellung von organischen Aminen für die Synthese organischer Isocyanatverbindungen bereitgestellt. Dabei gelten alle bevorzugten Ausführungsformen dieses Verfahrens zur Herstellung von organischen Aminen für die Synthese organischer Isocyanatverbindungen auch hier in diesem Verfahrensschritt als bevorzugt.

Zur Synthese von Phosgen kann beispielsweise industriell durch die Umsetzung von Chlorgas und Kohlenstoffmonoxid bei erhöhten Temperaturen an einem speziellen Aktivkohlekatalysator unter Aufwendung von Energie, z.B. zur Kühlung der Reaktionszone und zur Temperaturerhöhung, hergestellt werden. Hierfür werden im Industriemaßstab klassischerweise mit Aktivkohle bestückte Rohrreaktoren verwendet. Entsprechende Vorrichtungen und Verfahren zur Synthese von Phosgen sind dem Fachmann notorisch bekannt.

Für die Bereitstellung des gasförmigen Chlors für die Phosgensynthese ist dem Fachmann die Herstellung von Chlorgas aus elektrochemischer Oxidation nach der Salzsäure-Elektrolyse mit Gasdiffusionselektrode (auch als HCl ODC Elektrolyse-Verfahren bezeichnet (ODC steht für Oxygen Depleting Electrode, als eine ODC wird beispielsweise die sogenannte SVK (Sauerstoffverzehrkathode) eingesetzt); geeignete Elektrolysezelle vgl. US,6022,634 A, WO 03/31690 A1), die Herstellung von Chlorgas aus Salzsäure-Diaphragmaelektrolyse (vgl. EP 1 103 636 A1), die Herstellung von Chlorgas aus thermokatalytischer Gasphasenoxidation (vgl. WO 2012/025483 A2), sowie die Herstellung von Chlor aus Chloralkali-Elektrolyse (vgl. WO 2009/007366 A2) hinlänglich bekannt. Auf den Inhalt der vorgenannten, im Zusammenhang mit der Herstellung von Chlorgas zitierten Schriften wird ausdrücklich und vollumfänglich Bezug genommen. Es sind somit folgende Ausführungsformen des erfindungsgemäßen Verfahrens bevorzugt, worin das Chlorgas zumindest durch mindestens eine Chlorsynthese bereitgestellt wird, ausgewählt aus
A) einer oxidativen Umsetzung des abgetrennten und aufgereinigten Chlorwasserstoffs aus der Isocyanatherstellung in einer thermokatalytischen Gasphasenoxidation mit Sauerstoff zu Chlor und Wasser,
B) einer Umsetzung des aufgereinigten Chlorwasserstoffes aus der Isocyanatherstellung mit Wasser zu Salzsäure, ggf. der Reinigung und elektrochemische Oxidation der Salzsäure zu Chlor und ggf. Wasserstoff.

Das für die Synthese von Phosgen benötigte Chlor wird in einer bevorzugten Variante dieser Ausführungsform des Verfahrens elektrolytisch hergestellt, insbesondere durch elektrochemische Oxidation nach der Salzsäure-Elektrolyse mit Gasdiffusionselektrode, durch elektrochemische Oxidation nach der Salzsäure-Diaphragmaelektrolyse oder durch elektrochemische Oxidation nach der Chloralkali-Elektrolyse. Dabei ist es wiederum besonders bevorzugt, wenn die besagte elektrochemische Oxidation in der Elektrolyse jeweils unter Verwendung von aus regenerativer Energie erzeugtem elektrischem Strom durchgeführt wird, insbesondere aus regenerativer Energie in Form von Windkraft, Sonnenenergie oder Wasserkraft.

Das für die Synthese von Phosgen benötigte Kohlenmonoxid wird in einer weiteren bevorzugten Ausführungsform des Verfahrens aus CO₂ hergestellt, zumindest durch mindestens ein Verfahren, ausgewählt aus
A) der elektrochemischen Reduktion von CO₂ unter Einsatz von aus regenerativer Energie hergestellter elektrischer Energie,
B) einem Reformerprozess, worin Methan (bevorzugt Bio-Methan) und Wasserdampf unter Zusatz von mindestens CO₂, unter Zuführung von Wärmeenergie bei einer Temperatur von mindestens 500°C zu Kohlenmonoxid umgesetzt werden,
C) Umsetzung von Wasserstoff und CO₂ in einer reverse water-gas shift RWGS-Reaktionszone zu Kohlenmonoxid.

Der Fachmann kennt beispielsweise aus der Druckschrift WO 2021/069470 A Elektroden und eine Methode zur Ausführung einer elektrochemischen Reduktion von CO₂. Bevorzugterweise wird die elektrochemischen Reduktion von CO₂ nach einem Verfahren der WO 2021/069470 A durchgeführt. Auf diese Druckschrift wird hierin ausdrücklich und vollinhaltlich Bezug genommen. In diesem bevorzugten elektrolytischen Verfahren zu Herstellung von Kohlenmonoxid werden Kohlenmonoxid, ggf. Wasserstoff und Chlor durch elektrochemische Umsetzung von Kohlendioxid und Alkalichloridlösung erhalten. Dieses bevorzugte Elektrolyseverfahren ist dadurch gekennzeichnet, dass das Kohlendioxid an einer Gasdiffusionselektrode als Kathode in einer wässrigen Alkalichlorid-haltigen Lösung als Katholyt elektrochemisch reduziert und gleichzeitig Chlor aus einer wässrigen Alkalichlorid-haltigen Lösung als Anolyt anodisch erzeugt wird, wobei das im Katholyt gebildete Alkalisalz der Kohlensäure, ausgewählt aus Alkalicarbonat, Alkalihydrogencarbonat oder Mischungen daraus anschließend mit Chlorwasserstoff zu Kohlendioxid und Alkalichlorid umgesetzt wird und das hierbei freigesetzte Kohlendioxid in den Kathodenraum zur Gasdiffusionselektrode und das erzeugte Alkalichlorid wahlweise in den Anodenraum und/oder in den Kathodenraum zurückgeführt werden.

Ein geeigneter Reformerprozess, worin, Methan (bevorzugt Bio-Methan) und Wasserdampf unter Zusatz von mindestens CO₂, unter Zuführung von Wärmeenergie bei einer Temperatur von mindestens 500°C zu Kohlenmonoxid umgesetzt werden, wird in der PCT-Patentanmeldung mit der Anmeldenummer PCT/EP2022/052267 beschrieben, auf die ausdrücklich und vollinhaltlich Bezug genommen wird. Dieses Verfahren betrifft die Herstellung von Kohlenmonoxid aus Methan, Wasserdampf und CO₂ für die Darstellung von Phosgen zur Synthese von organischem Isocyanat, enthaltend zumindest die Schritte
Synthese von Kohlenmonoxid in einem Reformerprozess, worin Methan und Wasserdampf unter Zusatz von mindestens CO₂ unter Zuführung von Wärmeenergie bei einer Temperatur von mindestens 500°C zu Kohlenmonoxid-haltigem Produktgas umgesetzt wird,
Reinigung des aus vorgenannter Synthese erhaltenen Kohlenmonoxid-haltigen Produktgases, mindestens durch Abtrennung von CO₂ und gegebenenfalls zusätzlich durch mindestens eine Abtrennung ausgewählt aus der Abtrennung von Wasser, der Abtrennung von Wasserstoff oder einer Kombination davon, unter Erhalt von Kohlenmonoxid;
Bereitstellung von CO₂ für den besagten Zusatz zum vorgenannten Reformerprozess zumindest aus besagter Abtrennung von CO₂ des vorgenannten Reinigungsschritts,
mit der Maßgabe, dass
1) die zur Synthese von Kohlenmonoxid dem Reformerprozess zugeführte Wärmeenergie durch mindestens eine Methode bereitgestellt wird, ausgewählt aus (i) der Verbrennung von Brennstoff, der mittels regenerativer Energie erzeugten Wasserstoff enthält, (ii) der Verbrennung von Brennstoff, der Methan aus biologischer Quelle enthält, (iii) Umwandlung von aus regenerativer Energie erzeugter, elektrischer Energie in Wärme; oder
2) für die Bereitgestellung des der Synthese des vorgenannten Reformerprozesses zugesetzten CO₂ zusätzlich CO₂ aus einer weiteren CO₂-Quelle herangezogen wird; oder
3) eine Kombination der vorgenannten Möglichkeiten 1) und 2) gewählt wird.

Als Quelle von Methan kommt für den Reformerprozess besonders bevorzugt Methan aus biologischer Quelle in Frage. Unter "Methan aus biologischer Quelle" (auch als Bio-Methan" bezeichnet, versteht der Fachmann in Abgrenzung zu fossilem Methan solches Methan, welches technisch durch Methan-Gärung aus Biomasse gewonnen wird. Die Methan-Gärung ist bekanntermaßen der anaerobe Abbau organischer Stoffe durch Mikroorganismen. Methan aus biologischer Quelle wird beispielsweise in Biogasanlagen hergestellt, worin sowohl organische Abfälle als auch nachwachsende Rohstoffe entsprechend vergoren werden.

Im Rahmen einer besonders bevorzugten Ausführungsform des erfindungsgemäßen Verfahrens in seiner Ausführungsform mit Phosgenherstellung für die Isocyanatsynthese, wird das Kohlenmonoxid für die Phosgensynthese, insbesondere für die Phosgensynthese gemäß Schritt iv), aus CO₂ hergestellt, durch mindestens eine Umsetzung von Wasserstoff und CO₂ in einer reverse water-gas shift (RWGS) Reaktionszone zu Kohlenmonoxid. Ein solche Ausführungsform wird beispielsweise in dem Dokument WO 2021/089737 A beschrieben, auf das hierin ausdrücklich und vollinhaltlich Bezug genommen wird.

Eine "Reaktionszone" ist der Teil eines Reaktionsraumes, in dem eine chemische Reaktion, z.B. die reverse watergas shift Reaktion, abläuft. Ein "Reaktionsraum" ist ein Volumen, in dem die an einer chemischen Reaktion beteiligten Reaktionspartner zusammengebracht werden und in dem die chemische Reaktion stattfindet. Dies kann für eine chemische Reaktion beispielsweise das Volumen eines Gefäßes sein, in dem sich ein Edukt, z.B. im Falle einer RWGS-Reaktion Kohlendioxid, und dessen Reaktionspartner, im Falle einer RWGS-Reaktion Wasserstoff, gemeinsam befinden und in der Reaktionszone zur Reaktion gebracht werden.

In diesem besonders bevorzugten Verfahren werden zur Kohlenmonoxid-Herstellung ganz besonders bevorzugt mindestens folgende Schritte durchgeführt
Reinigung eines CO₂ Gasstroms von Nebenbestandteilen, insbesondere von Stickoxiden, Schwefelverbindungen, Staub, Wasser, Sauerstoff und HCl wahlweise mittels Adsorption, Gaswäsche oder katalytischer Behandlung unter Erhalt eines gereinigten Kohlendioxids,
Einspeisung von bereitgestelltem Wasserstoffgas zusammen mit dem gereinigten CO₂ Gasstrom in eine RWGS Reaktionszone und Umsetzung der Edukte nach dem Prinzip der RWGS Reaktion zu einem Produktgasgemisch aus Wasserdampf, CO und gegebenenfalls Nebenprodukten, insbesondere niedere Kohlenwasserstoffe, insbesondere bevorzugt Methan,
Abtrennung von nicht umgesetztem Kohlendioxid aus dem aus der Abtrennung erhaltenen Gasgemischs der RWGS Reaktion, insbesondere mittels Aminwäsche, und Rückführung des nicht umgesetzten Kohlendioxids in die RWGS Reaktion,
Abtrennung des in der RWGS Reaktion nicht umgesetzten Wasserstoffs aus dem nach der Abtrennung erhaltenen Gasgemisch von Kohlenmonoxid und Wasserstoff, insbesondere unter Verwendung einer Coldbox, und wahlweise Rückführung des Wasserstoffs in die RWGS Reaktion oder Einspeisung des Wasserstoffs in die Hydrierung der organischen Nitroverbindungen,
Einspeisung des verbliebenen Kohlenmonoxids aus der Abtrennung in die Phosgensynthese.

Die durch das erfindungsgemäße Verfahren hergestellten organischen Aminoverbindungen eignen sich wie zuvor beschrieben bevorzugt für die Synthese von organischem Isocyanat. Im Rahmen einer weiteren bevorzugten Ausführungsform des erfindungsgemäßen Verfahrens werden für die Synthese organischen Isocyanats zusätzlich mindestens folgende Schritte ausgeführt:
iv) Synthese von Phosgen aus zumindest Kohlenmonoxid und Chlorgas (8b);
v) Isocyanatherstellung von organischem Isocyanat durch Umsetzung zumindest des Phosgens mit der mindestens einen organischen Aminoverbindung, unter Freisetzung von Chlorwasserstoff,
wobei für die Herstellung der Kohlenmonoxids mindestens folgende Schritte durchgeführt werden:
Reinigung eines CO₂ Gasstroms von Nebenbestandteilen, insbesondere von Stickoxiden, Schwefelverbindungen, Staub, Wasser, Sauerstoff und HCl wahlweise mittels Adsorption, Gaswäsche oder katalytischer Behandlung unter Erhalt eines gereinigten Kohlendioxids,
Einspeisung von bereitgestelltem Wasserstoffgas aus Schritt ii) zusammen mit dem gereinigten CO₂ Gasstrom in eine RWGS Reaktionszone und Umsetzung der Edukte nach dem Prinzip der RWGS Reaktion zu einem Produktgasgemisch aus Wasserdampf, CO und gegebenenfalls Nebenprodukten, insbesondere niedere Kohlenwasserstoffe, insbesondere bevorzugt Methan,
Abtrennung von nicht umgesetztem Kohlendioxid aus dem aus der Abtrennung erhaltenen Gasgemischs der RWGS Reaktion, insbesondere mittels Aminwäsche, und Rückführung des nicht umgesetzten Kohlendioxids in die RWGS Reaktion,
Abtrennung des in der RWGS Reaktion nicht umgesetzten Wasserstoffs aus dem nach der Abtrennung erhaltenen Gasgemisch von Kohlenmonoxid und Wasserstoff, insbesondere unter Verwendung einer Coldbox, und wahlweise Rückführung des Wasserstoffs in die RWGS Reaktion oder Einspeisung des Wasserstoffs in die Hydrierung der organischen Nitroverbindungen,
Einspeisung des verbliebenen Kohlenmonoxids aus der Abtrennung in die Phosgensynthese.

Als CO₂-Quelle zur Bereitstellung von Kohlenmonoxid dienen für die zuvor genannten Schritte A) und/oder B) und/oder C) beispielsweise das bei einer Bereitstellung von Wärmeenergie für die Schritte B) oder C) emittierte CO₂, das bei einer Luftzerlegung für die Bereitstellung von Stickstoff für die Bereitstellung von Ammoniak emittierte CO₂ oder CO₂ aus einer externen CO₂-Quelle.

Als eine externe CO₂-Quelle, die CO₂ beiträgt, welches nicht durch das erfindungsgemäße Verfahren oder dessen Ausführungsformen emittiert wird. Eine externe CO₂ Quelle wäre z.B. das CO₂, das bei einer Zementherstellung, bei Verbrennung von Brennstoffen (z.B. einer Abfallverbrennung) im Abgas entsteht, oder CO_{2,} das aus der Luft gewonnenen wird. Dieses CO₂ aus einer externen CO₂-Quelle wird im Rahmen einer bevorzugten Ausführungsform des erfindungsgemäßen Verfahrens durch Absorption eines CO₂ -Anteils aus (i) Prozessgasen oder Abgasen, die aus mindestens einem Prozess ausgewählt aus Zementherstellung, H₂-Herstellung, Verbrennung ausgewählt wird und/oder (ii) aus Luft durch einleiten in Alkalilauge, zum Beispiel Kalilauge, erfolgen. Hierbei bildet sich Kaliumhydrogencarbonat, welches anschließend wieder zu CO₂ und Kalilauge thermisch zersetzt werden kann. Das dabei freigesetzte CO₂ wird dann dem erfindungsgemäßen Prozess zur Synthese von Kohlenmonoxid zugeführt.

Die RWGS Reaktion wird bevorzugt in Gegenwart mindestens eines Katalysators durchgeführt. Dieser ist besonders bevorzugt ausgewählt aus mindestens einer Verbindung aus der Gruppe:
(I) Mischmetalloxide der Formel A_{(1-w-x)}A'_{w}A"ₓB_{(1-y-z)}B'_{y}B"_{z}O_{3-delta}
   wobei hier gilt:
   A, A' und A" sind unabhängig voneinander ausgewählt aus der Gruppe: Mg, Ca, Sr, Ba, Li, Na, K, Rb, Cs, Sn, Sc, Y, La, Ce, Pr, Nd, Pm, Sm, Eu, Gd, Tb, Dy, Ho, Er, Tm. Yb, Tl , Lu, Ni, Co, Pb, Bi und/oder Cd; und
   B, B' und B" sind unabhängig voneinander ausgewählt aus der Gruppe: Cr, Mn, Fe, Bi, Cd, Co, Cu, Ni, Sn. AI, Ga, Sc, Ti, V, Nb, Ta, Mo, Pb. Hf, Zr, Tb. W, Gd. Yb, Mg, Li, Na, K, Ce und/oder Zn; und
   0 ≤ w ≤ 0.5; 0 ≤ x < 0.5; 0 ≤ y ≤ 0.5; 0 ≤ z ≤ 0.5 und - 1 ≤ delta ≤ I ;
(II) Mischmetalloxide der Formel A_{(1-w-x)}A'_{w}A"ₓB_{(1-y-z)}B'_{y}B"_{z}O_{3-delta} wobei hier gilt:
   A, A' und A" sind unabhängig voneinander ausgewählt aus der Gruppe: Mg, Ca, Sr, Ba. Li, Na, K, Rb, Cs, Sn, Sc, Y, La, Ce, Pr, Nd, Sm, Eu, Gd, Tb, Dy, Ho, Er, Tm, Yb, Tl , Lu, Ni, Co, Pb und/oder Cd; und
   B ist ausgewählt aus der Gruppe: Cr, Mn, Fe, Bi, Cd, Co, Cu, Ni, Sn, AI, Ga, Sc, Ti, V, Nb, Ta, Mo, Pb, Hf, Zr, Tb, W, Gd, Yb, Mg, Cd, Zn, Re, Ru, Rh, Pd, Os, Ir und/oder Pt; und
   B' ist ausgewählt aus der Gruppe: Re, Ru, Rh, Pd, Os, Ir und/oder Pt; und B" ist ausgewählt aus der Gruppe: Cr, Mn, Fe, Bi, Cd, Co, Cu, Ni, Sn, AI, Ga, Sc, Ti, V, Nb, Ta, Mo, Pb, Hf, Zr, Tb. W. Gd, Yb. Mg, Cd und/oder Zn; und
   0 ≤ w ≤ 0,5; 0 ≤ x ≤ 0,5; 0 ≤ y ≤ 0,5; 0 ≤ z ≤ 0.5 und - I ≤ delta ≤ 1 ;
(III) Mischungen von wenigstens zwei verschiedenen Metallen M1 und M2 auf einem Träger, welcher ein mit einem Metall M3 dotiertes Oxid von AI, Ce und/oder Zr umfasst;
   wobei hier gilt: M1 und M2 sind unabhängig voneinander ausgewählt aus der Gruppe: Re, Ru, Rh, Ir, Os, Pd und/oder Pt; und
   M3 ist ausgewählt aus der Gruppe: Sc, Y, La, Ce, Pr, Nd, Sm, Eu, Gd, Tb, Dy, Ho, Er, Tm, Yb und/oder Lu;
(IV) Mischmetalloxide der Formel LOₓ(M_{(y/z)}Al_{(2-y/z)}O₃)_{z}; wobei hier gilt:
   L ist ausgewählt aus der Gruppe: Na, K, Rb, Cs, Mg, Ca, Sr, Ba, Sc, Y, Sn, Pb, Pd, Mn, In, Tl, La, Ce, Pr, Nd, Sm, Eu, Gd, Tb, Dy, Ho, Er, Tm. Yb und/oder Lu; und
   M ist ausgewählt aus der Gruppe: Ti, Zr, Hf , V, Nb, Ta, Cr, Mo, W, Mn, Re, Fe, Ru, Os, Co, Rh. Ir, Ni, Pd. Pt. Zn, Cu, Ag und/oder Au; und
   1 < x ≤ 2; 0 < y ≤ 12; und 4 ≤ z ≤ 9;
(V) Mischmetalloxide der Formel LO(Al₂O₃)_{z}; wobei hier gilt:
   L ist ausgewählt aus der Gruppe: Na, K, Rb. Cs, Mg, Ca, Sr, Ba, Sc, Y, Sn, Pb, Mn, In, Tl, La, Ce, Pr, Nd, Sm, Eu, Gd, Tb, Dy, Ho, Er, Tm, Yb und/oder Lu; und
   4 ≤ z ≤ 9;
(VI) oxidischer Katalysator, der Ni und Ru umfasst.
(VII) Metall M1 und/oder wenigstens zwei verschiedene Metalle M1 und M2 auf und/oder in einem Träger, wobei der Träger
   ein Carbid, Oxycarbid, Carbonitrid, Nitrid, Borid, Silicid, Germanid und/oder Selenid der Metalle A und/oder B ist; wobei hier gilt:
   M1 und M2 sind unabhängig voneinander ausgewählt aus der Gruppe: Cr, Mn, Fe, Co, Ni, Re, Ru, Rh, Ir, Os, Pd, Pt, Zn, Cu, La, Ce, Pr, Nd, Sm, Eu, Gd, Tb, Dy, Ho, Er, Tm, Yb, und/oder Lu; und
   A und B sind unabhängig voneinander ausgewählt aus der Gruppe: Be, Mg, Ca, Sc, I i, V, Cr, Mn, Fe, Co, Ni, Y, Zr, Nb, Mo, Hf, Ta, W, La, Ce, Pr, Nd, Sm, Eu, Gd, Tb, Dy, Ho, Er, Tm, Yb, und/oder Lu;
und/oder
Reaktionsprodukte von (I), (II), (III), (IV), (V), (VI) und/oder (VII) in Gegenwart von Kohlendioxid, Wasserstoff, Kohlenmonoxid und/oder Wasser bei einer Temperatur von ≥ 700 °C.

Besonders bevorzugt ist ein erfindungsgemäßes Verfahren in der Ausführungsform mit Phosgenherstellung für die Isocyanatsynthese und einer Pyrolyse von organischem Material zur Herstellung der in Schritt i-2) eingesetzten organischen Verbindungen, in dem zur Bereitstellung von Kohlenmonoxid der Reststoff aus der Gasifizierung in einer Verbrennung in Kohlendioxid umgewandelt wird und das gegebenenfalls aufgereinigte Kohlendioxid in das Verfahren zur Bereitstellung von Kohlenmonoxid eingebracht wird.

Besonders bevorzugt ist daher eine Ausführungsform des erfindungsgemäßen Verfahrens, in der zusätzlich
mindestens eine in Schritt i-2) eingesetzte organische Verbindung eine aromatische, organische Verbindung ist, die als unmittelbares Verfahrensprodukt eines Verfahrens bereitgestellt wird, umfassend mindestens folgende Schritte:
   Pyrolyse von organischem Material, bei einer Temperatur von 600 bis 1000°C unter Erhalt von gasförmigem Pyrolysat und Pyrolyserückstand;
   Kontaktieren des gasförmigen Pyrolysates mit einem heterogen Katalysator unter Erhalt von Konversionsprodukt, enthaltend mindestens eine aromatische, organische Verbindung mit einem Molekulargewicht von weniger als 300 g/mol;
   Aufarbeitung des Konversionsproduktes unter Erhalt der besagten aromatischen, organischen Verbindung, insbesondere ausgewählt aus Benzol, Toluol oder Xylol;
wobei zusätzlich die folgenden Schritte durchgeführt werden:
   Gasifizierung des Pyrolyserückstandes aus der Pyrolyse von organischem Material zu mindestens Kohlenmonoxid, welches der Synthese von Phosgen zugeführt wird;
   Umwandlung des Reststoffes aus der Gasifizierung durch Verbrennung in Kohlendioxid und Einbringung des gegebenenfalls aufgereinigten Kohlendioxids in ein Verfahren zur Bereitstellung von Kohlenmonoxid für die Phosgensynthese, ausgewählt aus
      A) der elektrochemischen Reduktion von CO₂ unter Einsatz von aus regenerativer Energie hergestellter elektrischer Energie,
      B) einem Reformerprozess, worin Methan und Wasserdampf unter Zusatz von mindestens CO₂, unter Zuführung von Wärmeenergie bei einer Temperatur von mindestens 500°C zu Kohlenmonoxid umgesetzt werden,
      C) Umsetzung von Wasserstoff und CO₂ in einer reverse water-gas shift RWGS-Reaktionszone zu Kohlenmonoxid.

Ein weiterer Gegenstand der Erfindung ist eine Vorrichtung, die zur Herstellung organischer Aminoverbindungen für die Herstellung organischer Isocyanatverbindungen konfiguriert ist. Diese Vorrichtungskonfiguration sieht vor, dass der Vorrichtungsteil zur Bereitstellung von Salpetersäure mit einer Salpetersäure befüllt ist, die ein unmittelbares Verfahrensprodukt eines Verfahrens zur Salpetersäureherstellung ist, das mindestens folgende Schritte umfasst:
a) Bereitstellung von Wasserstoffgas und Sauerstoffgas durch Ausführung einer Wasserelektrolyse in einer Elektrolysevorrichtung unter Einsatz von aus regenerativer Energie hergestellter elektrischer Energie und Wasser;
b) Herstellung von Ammoniak durch Umsetzung von zumindest Stickstoff und Wasserstoffgas aus besagter Wasserelektrolyse in einer Ammoniaksynthese ;
c) Herstellung von Salpetersäure durch Umsetzung von zuvor hergestelltem Ammoniak in einer Salpetersäure-Herstellung zumindest mit Sauerstoff-haltigem Gas, bevorzugt mindestens ein Sauerstoff-haltiges Gas, ausgewählt aus Sauerstoff aus besagter Wasserelektrolyse, Luft oder Sauerstoff aus einer Luftzerlegung.

Im Einzelnen ist somit eine Vorrichtung erfindungsgemäß, die zur Herstellung organischer Aminoverbindungen konfiguriert ist, wobei die Vorrichtung mindestens eine Vorrichtung zur Bereitstellung von Wasserstoffgas, mindestens eine Vorrichtung zur Herstellung von organischen Nitroverbindungen, mindestens eine Vorrichtung zur Hydrierung von organischen Nitroverbindungen und mindestens eine Bereitstellung von Salpetersäure enthält, wobei
- die mindestens eine Vorrichtung zur Bereitstellung von Salpetersäure mindestens einen Auslass für Salpetersäure und mindestens einen Einlass für Salpetersäure und mindestens ein Volumen für Salpetersäure enthält;
- die mindestens eine Vorrichtung zur Herstellung von organischen Nitroverbindungen mindestens einen Reaktor, mindestens einen Einlass für organische Verbindungen, mindestens einen Einlass für Salpetersäure und mindestens einen Auslass für organische Nitroverbindungen enthält;
- die mindestens eine Vorrichtung zur Hydrierung von organischen Nitroverbindungen mindestens einen Reaktor, mindestens einen Einlass für organische Nitroverbindungen, mindestens einen Einlass für Wasserstoffgas und mindestens einen Auslass für organische Aminoverbindungen enthält;
dadurch gekennzeichnet, dass zur Konfiguration
i) das Volumen für Salpetersäure mit einer Salpetersäure befüllt ist, die ein unmittelbares Verfahrensprodukt eines Verfahrens zur Salpetersäureherstellung ist, das mindestens folgende Schritte umfasst:
   a) Bereitstellung von Wasserstoffgas und Sauerstoffgas durch Ausführung einer Wasserelektrolyse in einer Elektrolysevorrichtung unter Einsatz von aus regenerativer Energie hergestellter elektrischer Energie und Wasser;
   b) Herstellung von Ammoniak durch Umsetzung von zumindest Stickstoff und Wasserstoffgas aus besagter Wasserelektrolyse in einer Ammoniaksynthese;
   c) Herstellung von Salpetersäure durch Umsetzung von zuvor hergestelltem Ammoniak in einer Salpetersäure-Herstellung zumindest mit Sauerstoff-haltigem Gas , bevorzugt mindestens ein Sauerstoff-haltiges Gas, ausgewählt aus Sauerstoff aus besagter Wasserelektrolyse, Luft oder Sauerstoff aus einer Luftzerlegung;
ii) der Auslass für Salpetersäure der Vorrichtung zur Bereitstellung von Salpetersäure in Fluidverbindung mit dem Einlass für Salpetersäure der Vorrichtung zur Herstellung organischer Nitroverbindungen steht;
iii) der Auslass für organische Nitroverbindungen der Vorrichtung zur Herstellung organischer Nitroverbindungen in Fluidverbindung mit dem Einlass für organische Nitroverbindungen der Vorrichtung zur Hydrierung von organischen Nitroverbindungen steht;
iv) der Einlass für Wasserstoffgas der Vorrichtung zur Hydrierung von organischen Nitroverbindungen in Fluidverbindung mit dem Auslass für Wasserstoffgas der Vorrichtung zur Bereitstellung von Wasserstoffgas steht.

Das Volumen für Salpetersäure der Vorrichtung für die Bereitstellung von Salpetersäure kann beispielsweise das Volumen eines Lagerbehälters oder das Volumen einer Rohrleitung sein. Ist besagtes Volumen ein Lagerbehälter, so ist es im Rahmen einer weiteren Ausführungsform bevorzugt, wenn die Vorrichtung zusätzlich mindestens eine Entladevorrichtung für Transportbehälter vorsieht, die in Fluidverbindung mit dem Einlass für Salpetersäure der Vorrichtung zur Bereitstellung von Salpetersäure steht.

Im Rahmen einer besonders bevorzugten Ausführungsform der erfindungsgemäßen Vorrichtung kennzeichnet sich die Vorrichtung jedoch dadurch, dass das Volumen für Salpetersäure eine Rohrleitung ist und der Einlass für Salpetersäure der Vorrichtung zur Bereitstellung von Salpetersäure in Fluidverbindung mit dem Auslass einer Vorrichtung für die Herstellung von Salpetersäure steht.

Es hat sich darüber hinaus im Sinne der erfindungsgemäßen Verfahrens als bevorzugt herausgestellt, wenn die Bereitstellung von Wasserstoffgas für die Hydrierung organischer Nitroverbindungen unter Einsatz mindestens einer Elektrolysevorrichtung erfolgt, ausgewählt aus mindestens einer Elektrolysevorrichtung zur Darstellung von Wasserstoffgas aus der Gruppe, die gebildet wird aus einer Elektrolysevorrichtung für Wasser, einer Elektrolysevorrichtung für Alkalichlorid und einer Elektrolysevorrichtung für Salzsäure. Dabei kann beispielsweise die zur Bereitstellung von Wasserstoffgas für eine Ausführung der Salpetersäurebereitstellung eingebundene Elektrolysevorrichtung für Wasser entsprechend auch für die Bereitstellung von Wasserstoffgas für die Vorrichtung zur Hydrierung genutzt und entsprechend in die gesamte Vorrichtung eingebunden sein.

Die erfindungsgemäße Vorrichtung zur Bereitstellung von organischer Aminoverbindung ist bevorzugt ein Bestandteil einer Vorrichtung zur Bereitstellung organischer Isocyanatverbindungen und kennzeichnet sich in dieser Ausführungsform dadurch, dass der Auslass für organische Aminoverbindungen der Vorrichtung zur Hydrierung von organischen Nitroverbindungen in Fluidverbindung mit einem Einlass für organische Aminoverbindungen einer Vorrichtung zur Phosgenierung steht, wobei diese Vorrichtung zur Phosgenierung mindestens einen Einlass für Phosgen, mindestens einen Einlass für organische Aminoverbindungen und mindestens einen Auslass für organische Isocyanatverbindungen enthält.

Ein weiterer Gegenstand der Erfindung ist die Verwendung von Salpetersäure, die ein unmittelbares Verfahrensprodukt eines Verfahrens zur Salpetersäureherstellung ist, das mindestens folgende Schritte umfasst:
a) Bereitstellung von Wasserstoffgas und Sauerstoffgas durch Ausführung einer Wasserelektrolyse in einer Elektrolysevorrichtung unter Einsatz von aus regenerativer Energie hergestellter elektrischer Energie und Wasser;
b) Bereitstellung von Ammoniak durch Umsetzung von zumindest Stickstoff und Wasserstoffgas aus besagter Wasserelektrolyse (5) in einer Ammoniaksynthese;
c) Herstellung von Salpetersäure durch Umsetzung von zuvor hergestelltem Ammoniak in einer Salpetersäure-Herstellung zumindest mit Sauerstoff-haltigem Gas, bevorzugt mindestens ein Sauerstoff-haltiges Gas, ausgewählt aus Sauerstoff aus besagter Wasserelektrolyse, Luft oder Sauerstoff aus einer Luftzerlegung;
zur Herstellung organischer Aminoverbindung für die Synthese von organischer Isocyanatverbindung.

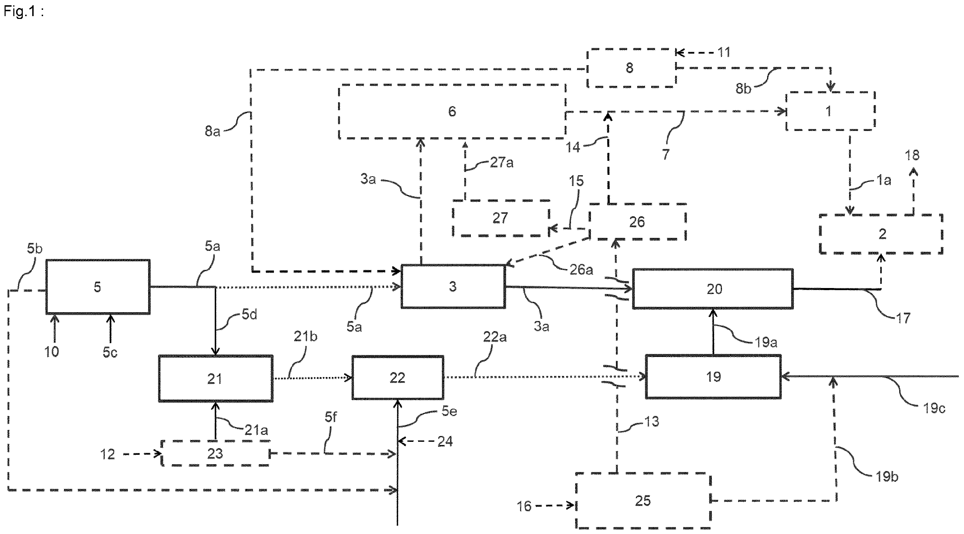

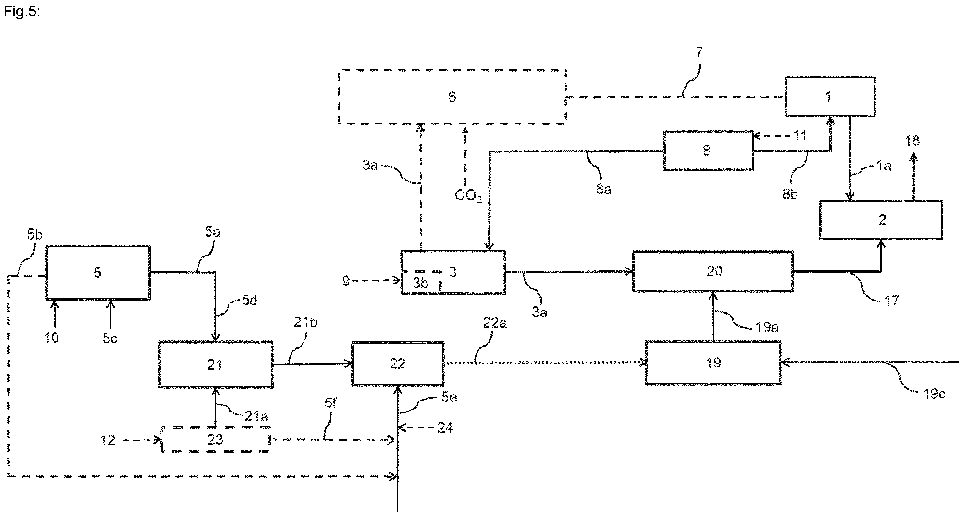

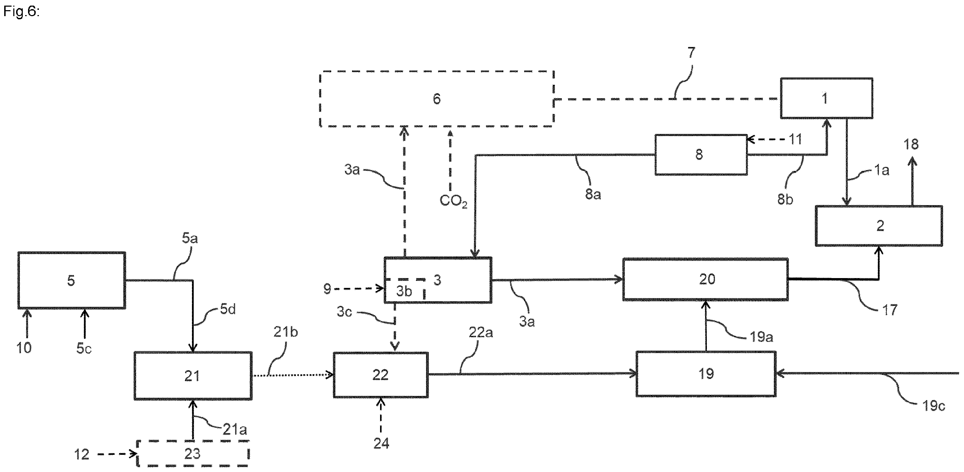

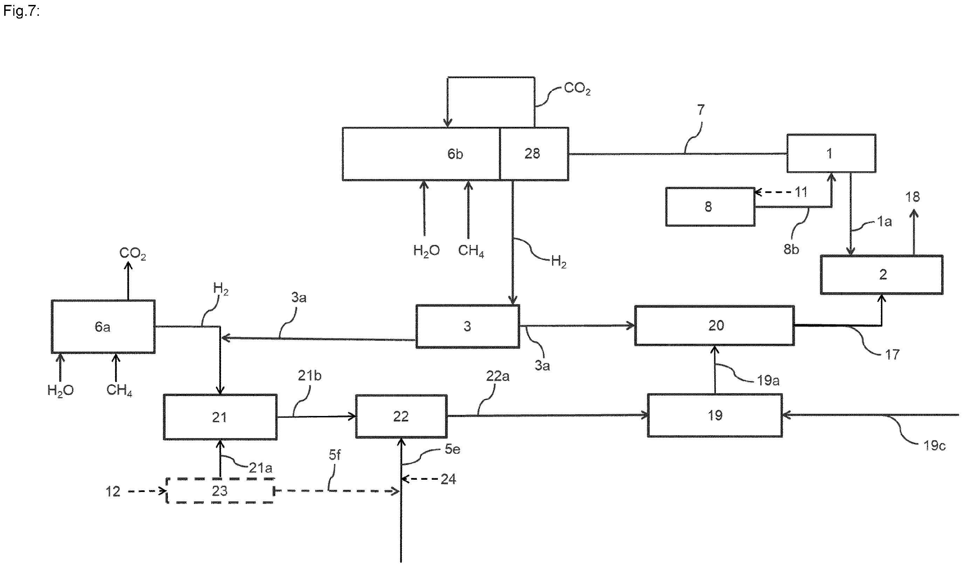

Die Erfindung wird nachstehend anhand der Figuren 1 bis 6 beispielhaft näher erläutert. Fig.7 stellt einen Prozess gemäß Stand der Technik dar. In den Figuren haben die folgenden Bezugszeichen die jeweils rechtsstehende Bedeutung:
- 1: Phosgensynthese
- 1a: Phosgen
- 2: Isocyanatherstellung
- 3: Bereitstellung von Wasserstoffgas (insbesondere aus der Wasserelektrolyse 5 als Wasserstoff 5a oder aus einer Wasserelektrolyse 3b)
- 3a: Wasserstoffgas entnommen aus der Bereitstellung von Wasserstoffgas
- 3b: Wasserelektrolyse
- 3c: O₂ entnommen aus der Wasserelektrolyse 3b der Bereitstellung von Wasserstoffgas 3
- 4: CO₂ Gas, gereinigt
- 5: Wasserelektrolyse
- 5a: Bereitstellung von Wasserstoffgas aus Wasserelektrolyse 5
- 5b: Sauerstoffgas aus der Wasserelektrolyse 5
- 5c: Wasser für die Wasserelektrolyse 5
- 5d: Wasserstoffgas aus der Wasserelektrolyse
- 5e: Sauerstoff-haltiges Gas für die HNO₃ Synthese (bevorzugt aus Wasserelektrolyse und/oder Luftzerlegung und/oder Luft)
- 5f: Sauerstoffgas (O₂) aus der Luftzerlegung
- 6: Reformerprozess mit RWGS (Reverse-Water Gas-Shift) zur CO-Herstellung aus Wasserstoff 3a und Kohlendioxid (nicht gezeigt, ggf. zusätzlich aus 27)
- 6a: Reformerprozess mit Wassergas-shift für H₂-Herstellung aus Methan und Wasser
- 6b: Reformerprozess in einem Steam-Methane-Reformer Reaktor für die CO-Herstellung aus Methan und Wasser
- 7: Kohlenmonoxid CO
- 8: Chlor-Synthese
- 8a: H₂ aus der Chlor-Herstellung
- 8b: Cl₂ aus der Chlor-Herstellung
- 9: Erneuerbare Energie für Wasserelektrolyse 3b
- 10: Erneuerbare Energie für Wasserelektrolyse 5
- 11: Erneuerbare Energie für die Chlor-Elektrolyse 8
- 12: Erneuerbare Energie für Luftzerlegung 23
- 13: Reststoff aus der Pyrolyse 25
- 14: CO aus der Gasifizierung 26
- 15: Reststoff aus Gasifizierung 26
- 16: organisches Material End of Life, bevorzugt Polyurethan-haltiges Material End of Life
- 17: Organisches Amin, z.B. Diaminotoluol
- 18: Organisches Isocyanat, z.B. Toluoldiisocyanat (TDI)
- 19: Herstellung von organischer Nitroverbindung
- 19a: Organische Nitroverbindung, z.B. Dinitrotoluol
- 19b: Zu nitrierende organische Verbindung aus der Pyrolyse 25
- 19c: Zu nitrierende organische Verbindung, z.B. Toluol
- 20: Hydrierung von Nitroverbindung
- 21: Ammoniaksynthese
- 21a: Stickstoff für Ammoniaksynthese
- 21b: Bereitstellung von Ammoniak
- 22: Salpetersäure-Herstellung
- 22a: Bereitstellung von Salpetersäure
- 23: N₂ Bereitstellung durch Luftzerlegung
- 24: Luft für die HNO₃ Herstellung
- 25: Pyrolyse von organischem Material, insbesondere von Polyurethanmaterial
- 26: Gasifizierung vor organischem Material, z.B. Rückstände aus der Pyrolyse 25
- 26a: H₂ aus Gasifizierung 26
- 27: Verbrennung
- 27a: CO₂ Gas, gereinigt aus Verbrennung 27
- 28: Aufbereitung von Prozessgas

Pfeile in den Figuren symbolisieren den Fluss von Stoffen, Energie oder Wärme zwischen Verfahrensschritten bzw. Vorrichtungsteilen, in denen die entsprechenden Verfahrensschritte ablaufen. Mit gestrichelten Linien gezeichnete Verfahrensmerkmale symbolisieren zusätzliche Merkmale weiterer, bevorzugter Ausführungsformen.

Pfeile mit gepunkteter Linie symbolisieren die Bereitstellung eines Stoffes, wobei die Bereitstellung durch Belieferung von einem Verfahrensschritt zum nächsten in einem abgeschlossenen Transportbehälter oder durch einen Stoffstrom über eine Fluidverbindung erfolgen kann. Pfeile mit durchgezogener Linie, die zwei Verfahrensschritte verbinden, symbolisieren einen Stoffstrom des bezeichneten Stoffes, der durch eine Fluidverbindung in Pfeilrichtung zwischen den durch den besagten Pfeil verbundenen Verfahrensschritten transportiert wird.

In Fig. 1 wird beispielhaft eine Herstellung organischer Aminoverbindung durch Hydrierung von organischer Nitroverbindung 20 illustriert, in der zunächst organische Nitroverbindungen 19a und Wasserstoff 3a aus einer Bereitstellung von Wasserstoff 3, zu organischen Aminoverbindungen 17 umgesetzt werden, wobei die hergestellten organischen Nitroverbindungen 19a in einer Herstellung von organischen Nitroverbindungen 19 hergestellt werden, in dem organische Verbindungen 19c und Salpetersäure umgesetzt werden und die dafür bereitgestellte Salpetersäure 22a ein unmittelbares Verfahrensprodukt einer Herstellung von Salpetersäure 22 ist, in welcher der aus Wasserstoff, der aus einer Wasserelektrolyse 5 unter Einsatz von Wasser 5c und regenerativer Energie 10 hergestellter wurde, und aus (bevorzugt aus einer Luftzerlegung 23 stammender) Stickstoff 21a bereitgestellter Ammoniak 21b mit sauerstoffhaltigem Gas 5e (wie beispielsweise Luft 24 oder Sauerstoff 5b aus der Wasserelektrolyse 5 oder Sauerstoff 5f aus der Luftzerlegung 23) zu Salpetersäure umgesetzt wird. Der bereitgestellte Ammoniak 21b sowie die bereitgestellte Salpetersäure 22a werden über die entsprechenden Verfahren bevorzugt am gleichen Ort wie die organischen Aminoverbindungen hergestellt und jeweils über eine Fluidverbindung als Stoffstrom für die jeweiligen weiteren Umsetzungen bereitgestellt. In diesem Fall kann der überschüssige Wasserstoff 5a aus der Wasserelektrolyse 5 zur Bereitstellung von Wasserstoff 3 für die Einbringung von Wasserstoff 3a in die Hydrierung von Nitroverbindungen 20 genutzt und über eine Fluidverbindung eingebracht werden.

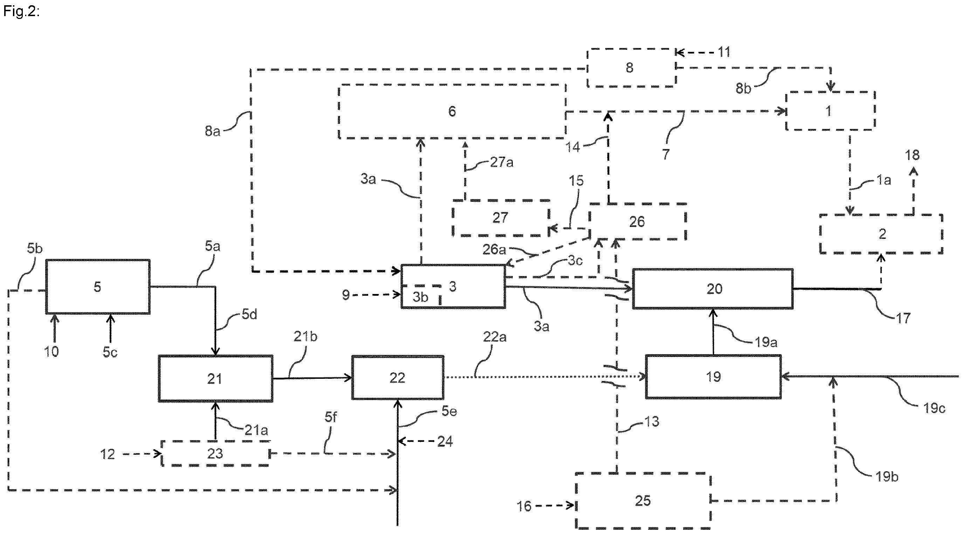

Gemäß Fig.1 ist ebenfalls die Variante möglich, die Salpetersäure dezentral nach den vorgenannten Verfahren herstellen zu lassen, um sich diese in einem Transportbehälter gemäß Bereitstellung von Salpetersäure 22a für die Herstellung organischer Nitroverbindung 19 liefern zu lassen. Diese Variante wird in Fig. 2 illustriert, worin der Ammoniak zur Bereitstellung von Ammoniak 21b über eine Fluidverbindung zur Herstellung von Salpetersäure 22 bereitgestellt und eingebracht wird.

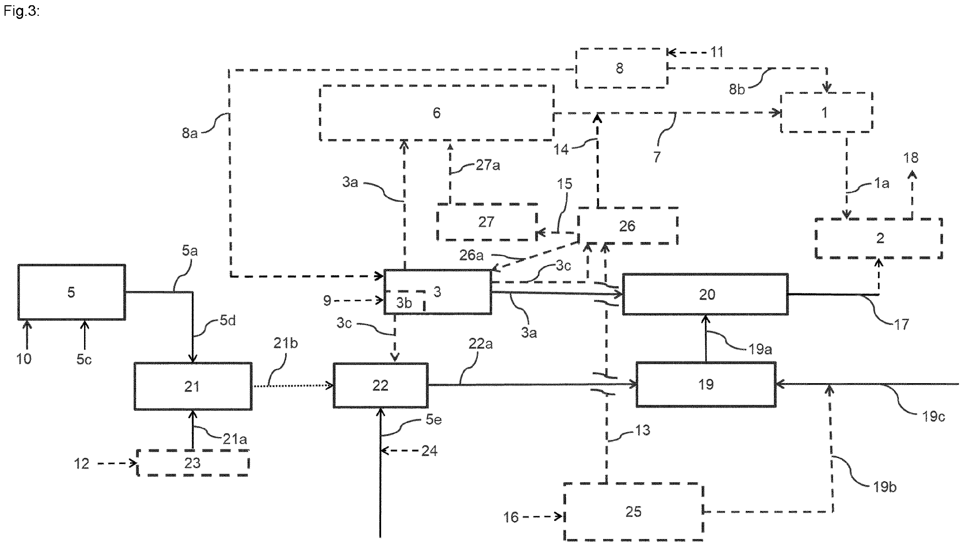

Allerdings ist es gemäß Fig.1 ebenso möglich und wie in Fig.3 illustriert, den Ammoniak beispielsweise dezentral nach vorgenanntem Verfahren herstellen zu lassen, um sich damit zur Bereitstellung von Ammoniak 21b in einem Transportbehälter beliefern zu lassen, mit dem Ziel, diesen Ammoniak in der Bereitstellung von Salpetersäure 22 zu Salpetersäure umzusetzen, die dann in die Herstellung von organischen Nitroverbindungen 19 über eine Fluidverbindung bereitgestellt und eingebracht wird.

Das in Fig.1 illustrierte Verfahren zur Herstellung organischer Aminoverbindungen lässt sich in eine Umsetzung dieser organischen Aminoverbindungen zu organischen Isocyanatverbindungen einbinden. Hierbei wird die aus der Hydrierung organischer Nitroverbindung 20 erhaltene organische Aminoverbindung 17 in einer Phosgenierung 2 eingebracht und mit Phosgen 1a zu organischer Isocyanatverbindung 18 umgesetzt. Das dafür genutzte Phosgen 1a wird in einer Phosgenherstellung 1 aus Chlor 8b und Kohlenmonoxid 7 hergestellt, wobei das Kohlenmonoxid 7 durch einem Reformerprozess mit RWGS 6 aus Wasserstoff 3a und Kohlendioxid (Zufuhr nicht eingezeichnet) hergestellt wird. Der Wasserstoff 3a stammt aus der Bereitstellung von Wasserstoffgas 3, bevorzugt aus einer Wasserelektrolyse 3b von Wasser mit Strom aus regenerativer Energie 9 im Falle von Fig. 2 und 3 bei dezentraler Bereitstellung von Ammoniak bzw. Salpetersäure. Bei Bereitstellung von Ammoniak und Salpetersäure über eine Fluidverbindung, wird zur Bereitstellung von Wasserstoffgas 3a bevorzugt die Wasserelektrolyse 5 genutzt.

Das Chlor 8b wird gemäß Fig. 1 bis 3 durch eine Chloralkalielektrolyse 8 unter Einsatz von Strom aus regenerativer Energie 11 bereitgestellt, wobei der dabei gegebenenfalls gebildete Wasserstoff 8a zur Bereitstellung von Wasserstoff 3 abgeführt und für die Hydrierung organischer Nitroverbindung 20 genutzt wird.

In den Fig.1 bis 3 wird bevorzugt zusätzlich Kohlenmonoxid 14 aus einer Gasifizierung von organischem Material 26, besonders bevorzugt von Rückstand 13 aus der Pyrolyse von organischem Material 25, hergestellt. Für die Gasifizierung 26 wir bevorzugt der Sauerstoff 3c aus der Wasserelektrolyse (3b) genutzt. Die Rückstände 15 aus der Gasifizierung 26 werden wiederum bevorzugt in einer Verbrennung zu Kohlendioxid umgesetzt, wobei Sauerstoff aus der 3c aus der Wasserlektrolyse (3b) genutzt wird. Das erhaltene Kohlendioxid wird gereinigt und als gereinigtes Kohlendioxid 27a dem Reformerprozess mit RWGS 6 zugeführt wird. Der Wasserstoff 26a aus der Gasifizierung 26 wird der Bereitstellung von Wasserstoff 3 zur Verfügung gestellt.

Gemäß Fig.1 bis 3 können die organischen Verbindungen 19c für die Nitrierung organischer Verbindungen 19 zumindest teilweise aus einer Pyrolyse 25 von organischem Abfallmaterial 16, bevorzugt Kunststoff-Abfall, insbesondere Polyurethanmaterial-Abfall, stammen.

Fig.4 illustriert den Gesamtprozess des erfindungsgemäßen Beispiels 1 (siehe dort).

Fig. 5 und 6 stellen Varianten des Beispiels 1 der Fig. 4 dar, in denen lediglich analog zu Fig.2 bzw. zu Fig.3 der Ammoniak bzw. die Salpetersäure dezentral nach vorgenanntem Verfahren bereitgestellt werden.

Fig. 7 illustriert das Beispiel 2 gemäß Stand der Technik.

Als weitere Ausführungsformen der Erfindung gelten folgende Aspekte I bis XXVI:
I. Verfahren zur Herstellung von organischer Aminoverbindung für die Synthese organischer Isocyanatverbindungen, enthaltend mindestens die Schritte
   i) Bereitstellung mindestens einer organischen Nitroverbindung (19a) als Verfahrensprodukt eines Verfahrens zur Synthese (19) mindestens einer organischen Nitroverbindung (19a), das mindestens folgende Schritte umfasst:
      i-1) Bereitstellung von Salpetersäure (22a) als Verfahrensprodukt eines Verfahrens zur Salpetersäureherstellung, das mindestens folgende Schritte umfasst:
         a) Bereitstellung von Wasserstoffgas (5d) und Sauerstoffgas (5b) durch Ausführung einer Wasserelektrolyse (5) in einer Elektrolysevorrichtung unter Einsatz von aus regenerativer Energie hergestellter elektrischer Energie (10) und Wasser (5c);
         b) Bereitstellung von Ammoniak (21b) als Verfahrensprodukt einer Umsetzung von zumindest Stickstoff (21a) und Wasserstoffgas (5d) aus besagter Wasserelektrolyse (5) in einer Ammoniaksynthese (21);
         c) Herstellung von Salpetersäure (22b) durch Umsetzung von zuvor bereitgestelltem Ammoniak (21b) in einer Salpetersäure-Herstellung (22) zumindest mit Sauerstoff-haltigem Gas (5e);
      i-2) Synthese (19) mindestens einer organischen Nitroverbindung (19a), bevorzugt mindestens einer aromatischen organischen Nitroverbindung (19a), zumindest durch Nitrierung mindestens einer organischen Verbindung (19b, 19c), bevorzugt mindestens einer aromatischen organischen Verbindung (19b, 19c), mit mindestens der besagten bereitgestellten Salpetersäure (22a);
   ii) Bereitstellung (3) von Wasserstoffgas (3a), bevorzugt durch Ausführung einer Wasserelektrolyse in einer Elektrolysevorrichtung (3b) unter Einsatz von aus regenerativer Energie (9) hergestellter elektrischer Energie;
   iii) Einbringung der mindestens einen bereitgestellten organischen Nitroverbindung (19a) und des bereitgestellten Wasserstoffgases in eine Hydrierung (20) und anschließende Hydrierung (20) durch Reduktion von Nitrogruppen der besagten mindestens einen organischen Nitroverbindung (19a) über deren Umsetzung mit besagtem Wasserstoffgas (3a) unter Erhalt mindestens einer organischen Aminoverbindung (17), bevorzugt mindestens einer aromatischen organischen Aminoverbindung (17).
II. Verfahren gemäß Aspekt I, dadurch gekennzeichnet, dass die organische Nitroverbindung (19a) als Verfahrensprodukt besagter Synthese (19) des Schritts i-2) in Form eines Stoffstromes direkt über eine Fluidverbindung aus der besagten Synthese (19) über eine gegebenenfalls notwendige Zwischenlagerung in mindestens einem Lagerbehälter bereitgestellt wird, wobei die besagte Synthese (19), der gegebenenfalls notwendige mindestens eine Lagerbehälter und die Hydrierung (20) gemäß Schritt iii) miteinander in Fluidverbindung stehen.
III. Verfahren gemäß Aspekt I, dadurch gekennzeichnet, dass die organische Nitroverbindung (19a) des Schritts i-2) als Verfahrensprodukt der besagten Synthese (19) in einem beweglichen, abgeschlossenem Transportbehälter bereitgestellt wird, gegebenenfalls daraus zur Zwischenlagerung in Lagerbehälter überführt wird, wobei die besagte Synthese (19) und die Hydrierung (20) gemäß Schritt iii) nicht miteinander in Fluidverbindung stehen.
IV. Verfahren gemäß einem der Aspekte I bis III, dadurch gekennzeichnet, dass in Schritt i-1) die Salpetersäure (22b) als Verfahrensprodukt besagter Salpetersäureherstellung (22) in Form eines Stoffstromes direkt über eine Fluidverbindung aus der besagten Salpetersäureherstellung (22) über eine gegebenenfalls notwendige Zwischenlagerung in mindestens einem Lagerbehälter bereitgestellt wird, wobei die besagte Salpetersäureherstellung (22), der gegebenenfalls notwendige mindestens eine Lagerbehälter und die Synthese (19) gemäß Schritt i-2) über eine Fluidverbindung miteinander verbunden sind.
V. Verfahren gemäß Aspekt IV, dadurch gekennzeichnet, dass in Schritt i-1) der Ammoniak (21b) als Verfahrensprodukt der besagten Herstellung von Ammoniak (21) in einem beweglichen, abgeschlossenem Transportbehälter bereitgestellt wird, gegebenenfalls daraus zur Zwischenlagerung in Lagerbehälter überführt wird,
   wobei die besagte Herstellung von Ammoniak (21) und die Salpetersäureherstellung (22) nicht miteinander in Fluidverbindung stehen.
VI. Verfahren gemäß Aspekt I bis III, dadurch gekennzeichnet, dass in Schritt i-1) die Salpetersäure (22b) als Verfahrensprodukt der besagten Salpetersäureherstellung (22) in einem beweglichen, abgeschlossenem Transportbehälter bereitgestellt wird, gegebenenfalls daraus zur Zwischenlagerung in Lagerbehälter überführt wird,
   wobei die besagte Salpetersäureherstellung (22) und die Synthese (19) gemäß Schritt ii) nicht miteinander in Fluidverbindung stehen.
VII. Verfahren nach einem der Aspekte I bis VI, dadurch gekennzeichnet, dass die in Schritt i-1) bereitgestellte Salpetersäure (22a) ein Verfahrensprodukt einer Salpetersäureherstellung (22) ist, in der der zur Herstellung von Ammoniak (21b) in Schritt b) genutzte Stickstoff (21a) durch eine Luftzerlegung (23) bereitgestellt wird.
VIII. Verfahren nach einem der Aspekte I bis VII, dadurch gekennzeichnet, dass die in Schritt i-1) bereitgestellte Salpetersäure (22a) ein Verfahrensprodukt einer Salpetersäureherstellung (22) ist, in der für die Oxidationsreaktion des Schritts c) mindestens ein Sauerstoff-haltiges Gas (5e), ausgewählt aus Sauerstoff (5b) aus einer Wasserelektrolyse (5, 3b), Luft (24) oder Sauerstoff (5f) aus einer Luftzerlegung (23) eingesetzt wird.
IX. Verfahren nach einem der Aspekte VII oder VIII, dadurch gekennzeichnet, dass die in Schritt i-1) bereitgestellte Salpetersäure (22a) ein Verfahrensprodukt einer Salpetersäureherstellung (22) ist, in der für die Oxidationsreaktion des Schritts c) Sauerstoff für das Sauerstoff-haltig Gas (5f) durch eine Luftzerlegung (23), insbesondere eine Luftzerlegung (23) die ebenfalls Stickstoff (21a) für Schritt b) gemäß Aspekt 5 liefert, bereitgestellt wird.
X. Verfahren nach einem der Aspekte I bis IX, dadurch gekennzeichnet, dass der in Schritt ii) bereitgestellte Wasserstoff mindestens durch eine Elektrolyse von Wasser (3b) unter Einsatz von elektrischer Energie (9) hergestellt wird, wobei die Bereitstellung elektrischer Energie (9) als regenerative Energie wahlweise Windkraft, Sonnenenergie, Wasserkraft oder Mischungen daraus, eingesetzt wird.
XI. Verfahren nach einem der vorangehenden Aspekte, dadurch gekennzeichnet, dass die in Schritt i-2) eingesetzte organische Verbindung (19b, 19c) ausgewählt wird aus Benzol oder Toluol.
XII. Verfahren nach einem der vorangehenden Aspekte, dadurch gekennzeichnet, dass mindestens eine in Schritt i-2) eingesetzte organische Verbindung (19c) eine aromatische, organische Verbindung (19b) ist, die als unmittelbares Verfahrensprodukt eines Verfahrens bereitgestellt wird, umfassend mindestens folgende Schritte:
   Pyrolyse (25) von organischem Material, bei einer Temperatur von 600 bis 1000°C unter Erhalt von gasförmigem Pyrolysat und Pyrolyserückstand (13);
   Kontaktieren des gasförmigen Pyrolysates mit einem heterogen Katalysator unter Erhalt von Konversionsprodukt, enthaltend mindestens eine aromatische, organische Verbindung mit einem Molekulargewicht von weniger als 300 g/mol;
   Aufarbeitung des Konversionsproduktes unter Erhalt der besagten aromatischen, organischen Verbindung (19b), insbesondere ausgewählt aus Benzol, Toluol oder Xylol.
XIII. Verfahren nach Aspekt XII, dadurch gekennzeichnet, dass der bei der Pyrolyse (25) anfallende Pyrolyserückstand (13) in einer Gasifizierung (26) unter partieller Oxidation zu Kohlenmonoxid (14), Wasserstoff (26a) und Reststoff (15) umgewandelt wird und das gebildete Kohlenmonoxid (14) nach optionaler Aufarbeitung in die Phosgensynthese (1) und der Wasserstoff (26a) in die Bereitstellung von Wasserstoff (3) eingebracht wird.
XIV. Verfahren nach einem der vorangehenden Aspekte, dadurch gekennzeichnet, dass die in Schritt iii) erhaltene organische Aminoverbindung (17) ausgewählt wird aus Toluylendiamin (TDA), Methylendi(phenylamin) (MDA) (wiederum bevorzugt ausgewählt aus Diphenylmethan-2,2'-diamin, Diphenylmethan-2,4'-diamin, Diphenylmethan-4,4'-diamin oder Mischungen daraus) oder Mischungen daraus.
XV. Verfahren nach einem der vorangehenden Aspekte, dadurch gekennzeichnet, dass für die Synthese organischen Isocyanats zusätzlich mindestens folgende Schritte ausgeführt werden:
   iv) Synthese (1) von Phosgen (1a) aus zumindest Kohlenmonoxid (7) und Chlorgas (8b);
   v) Isocyanatherstellung (2) von organischem Isocyanat (18) durch Umsetzung zumindest des Phosgens (1a) mit der mindestens einen organischen Aminoverbindung (17), unter Freisetzung von Chlorwasserstoff.
XVI. Verfahren zur Synthese organischen Isocyanats (18), enthaltend mindestens die Schritte
   Bereitstellung mindestens einer organischen Aminoverbindung (17) als Verfahrensprodukt eines Verfahrens nach einem der Aspekte I bis XIV;
   Synthese (1) von Phosgen (1a) aus zumindest Kohlenmonoxid (7) und Chlorgas (8b);
   Isocyanatherstellung (2) von organischem Isocyanat (18) durch Umsetzung zumindest des Phosgens (1a) mit der mindestens einen organischen Aminoverbindung (17), unter Freisetzung von Chlorwasserstoff.
XVII. Verfahren nach einem der Aspekte XV oder XVI, dadurch gekennzeichnet, dass das Kohlenmonoxid (7) aus CO₂ hergestellt wird, zumindest durch mindestens ein Verfahren (6), ausgewählt aus
   A) der elektrochemische Reduktion von CO₂ unter Einsatz von aus regenerativer Energie hergestellter elektrischer Energie,
   B) einem Reformerprozess, worin Bio-?-Methan und Wasserdampf unter Zusatz von mindestens CO₂, unter Zuführung von Wärmeenergie bei einer Temperatur von mindestens 500°C zu Kohlenmonoxid umgesetzt werden,
   C) Umsetzung von Wasserstoff und CO₂ in einer reverse water-gas shift RWGS-Reaktionszone zu Kohlenmonoxid.
XVIII. Verfahren nach Aspekt XVII, dadurch gekennzeichnet, dass der Reststoff (15) aus der Gasifizierung (26) gemäß Aspekt 12 in einer Verbrennung (27) in Kohlendioxid umgewandelt wird und das gegebenenfalls aufgereinigte Kohlendioxid in das Verfahren (6) zur Bereitstellung von Kohlenmonoxid eingebracht wird.
XIX. Verfahren nach einem der Aspekte XIII bis XVIII, dadurch gekennzeichnet, dass Chlorgas (8b) zumindest durch mindestens eine Chlorsynthese (8) bereitgestellt wird, ausgewählt aus
   A) einer oxidativen Umsetzung des abgetrennten und aufgereinigten Chlorwasserstoffs aus der Isocyanatherstellung (2) in einer thermokatalytischen Gasphasenoxidation mit Sauerstoff zu Chlor und Wasser,
   B) einer Umsetzung des Chlorwasserstoffes aus der Isocyanatherstellung (2) mit Wasser zu Salzsäure und elektrochemische Oxidation der Salzsäure zu Chlor (8b) und ggf. Wassertoff (8a).
XX. Verwendung von Salpetersäure, die ein unmittelbares Verfahrensprodukt eines Verfahrens zur Salpetersäureherstellung ist, das mindestens folgende Schritte umfasst:
   a) Bereitstellung von Wasserstoffgas (5d) und Sauerstoffgas (5b) durch Ausführung einer Wasserelektrolyse (5) in einer Elektrolysevorrichtung unter Einsatz von aus regenerativer Energie hergestellter elektrischer Energie (10) und Wasser (5c);
   b) Bereitstellung von Ammoniak (21b) durch Umsetzung von zumindest Stickstoff (21a) und Wasserstoffgas (5d) aus besagter Wasserelektrolyse (5) in einer Ammoniaksynthese (21);
   c) Herstellung von Salpetersäure (22b) durch Umsetzung von zuvor hergestelltem Ammoniak (21b) in einer Salpetersäure-Herstellung (22) zumindest mit Sauerstoff-haltigem Gas (5e), bevorzugt mindestens ein Sauerstoff-haltiges Gas, ausgewählt aus Sauerstoff (5b) aus besagter Wasserelektrolyse (5), Luft (24) oder Sauerstoff (5f) aus einer Luftzerlegung (23);
   zur Herstellung organischer Aminoverbindung für die Synthese von organischer Isocyanatverbindung.
XXI. Vorrichtung konfiguriert zur Herstellung organischer Aminoverbindungen, wobei die Vorrichtung mindestens eine Vorrichtung zur Bereitstellung von Wasserstoffgas, mindestens eine Vorrichtung zur Herstellung von organischen Nitroverbindungen, mindestens eine Vorrichtung zur Hydrierung von organischen Nitroverbindungen und mindestens eine Bereitstellung von Salpetersäure enthält, wobei
   - die mindestens eine Vorrichtung zur Bereitstellung von Salpetersäure mindestens einen Auslass für Salpetersäure und mindestens einen Einlass für Salpetersäure und mindestens ein Volumen für Salpetersäure enthält;
   - die mindestens eine Vorrichtung zur Herstellung von organischen Nitroverbindungen mindestens einen Reaktor, mindestens einen Einlass für organische Verbindungen, mindestens einen Einlass für Salpetersäure und mindestens einen Auslass für organische Nitroverbindungen enthält;
   - die mindestens eine Vorrichtung zur Hydrierung von organischen Nitroverbindungen mindestens einen Reaktor, mindestens einen Einlass für organische Nitroverbindungen, mindestens einen Einlass für Wasserstoffgas und mindestens einen Auslass für organische Aminoverbindungen enthält;
   dadurch gekennzeichnet, dass
   i) der Lagerbehälter der Bereitstellung von Salpetersäure mit einer Salpetersäure befüllt ist, die ein unmittelbares Verfahrensprodukt eines Verfahrens zur Salpetersäureherstellung ist, das mindestens folgende Schritte umfasst:
      a) Bereitstellung von Wasserstoffgas (5d) und Sauerstoffgas (5b) durch Ausführung einer Wasserelektrolyse (5) in einer Elektrolysevorrichtung unter Einsatz von aus regenerativer Energie hergestellter elektrischer Energie (10) und Wasser (5c);
      b) Herstellung von Ammoniak (21b) durch Umsetzung von zumindest Stickstoff (21a) und Wasserstoffgas (5d) aus besagter Wasserelektrolyse (5) in einer Ammoniaksynthese (21);
      c) Herstellung von Salpetersäure (22b) durch Umsetzung von zuvor hergestelltem Ammoniak (21b) in einer Salpetersäure-Herstellung (22) zumindest mit Sauerstoff-haltigem Gas (5e), bevorzugt mindestens ein Sauerstoff-haltiges Gas, ausgewählt aus Sauerstoff (5b) aus besagter Wasserelektrolyse (5), Luft (24) oder Sauerstoff (5f) aus einer Luftzerlegung (23);
   ii) der Auslass für Salpetersäure der Vorrichtung zur Bereitstellung von Salpetersäure in Fluidverbindung mit dem Einlass für Salpetersäure der Vorrichtung zur Herstellung organischer Nitroverbindungen steht;
   iii) der Auslass für organische Nitroverbindungen der Vorrichtung zur Herstellung organischer Nitroverbindungen in Fluidverbindung mit dem Einlass für organische Nitroverbindungen der Vorrichtung zur Hydrierung von organischen Nitroverbindungen steht;
   iv) der Einlass für Wasserstoffgas der Vorrichtung zur Hydrierung von organischen Nitroverbindungen in Fluidverbindung mit dem Auslass für Wasserstoffgas der Vorrichtung zur Bereitstellung von Wasserstoffgas steht.
XXII. Vorrichtung nach Aspekt XXI, dadurch gekennzeichnet, dass die Vorrichtung zur Bereitstellung von Wasserstoffgas (3) das Wasserstoffgas aus mindestens einer Elektrolyse zur Darstellung von Wasserstoffgas bezieht, ausgewählt aus einer Elektrolyse von Wasser, einer Elektrolyse von Alkalichlorid, einer Elektrolyse von Salzsäure oder aus mehreren der vorgenannten Elektrolysen.
XXIII. Vorrichtung nach Aspekt XXI oder XXII, dadurch gekennzeichnet, dass das Volumen für Salpetersäure der Vorrichtung zur Bereitstellung von Salpetersäure ein das Volumen eines Lagerbehälters oder das Volumen einer Rohrleitung ist.
XXIV. Vorrichtung nach Aspekt XXIII, dadurch gekennzeichnet, dass das Volumen für Salpetersäure ein Lagerbehälter ist und der Einlass für Salpetersäure der Vorrichtung zur Bereitstellung von Salpetersäure in Fluidverbindung mit einer Entladevorrichtung für Transportbehälter steht.
XXV. Vorrichtung nach Aspekt XXIII, dadurch gekennzeichnet, dass das Volumen für Salpetersäure eine Rohrleitung ist und der Einlass für Salpetersäure der Vorrichtung zur Bereitstellung von Salpetersäure in Fluidverbindung mit dem Auslass einer Vorrichtung für die Herstellung von Salpetersäure steht.
XXVI. Vorrichtung nach einem der Aspekte XXI bis XXV, dadurch gekennzeichnet, dass der Auslass für organische Aminoverbindungen der Vorrichtung zur Hydrierung von organischen Nitroverbindungen in Fluidverbindung mit einem Einlass für organische Aminoverbindungen einer Vorrichtung zur Phosgenierung steht, wobei diese Vorrichtung zur Phosgenierung mindestens einen Einlass für Phosgen, mindestens einen Einlass für organische Aminoverbindungen und mindestens einen Auslass für organische Isocyanatverbindungen enthält.

### Beispiele

Folgende Prozessparameter ergeben sich aus den Daten der einzelnen Prozessschritte als Stoffstrom des Gesamtprozesses:

### Bespiel 1 - Stand der Technik (Fig.7)

Toluol-di-isocyanat (TDI) wird gemäß dem Stand der Technik wie folgt hergestellt. Zur Illustration des Prozesses dient Fig.7.

Kohlenmonoxid (CO) 7 und Wasserstoff (H₂) werden über einen Steam-Methane-Reforming Prozess 6b (SMR) hergestellt. Hierzu werden 6,89 t/h Methan aus Erdgas einem SMR Reaktor 6b sowie 19,0 t/h Wasser zugeführt. In einem internem Kreislauf werden aus der Prozessgasaufbereitung 28, die dem SMR Reaktor (6) nachgeschaltet ist, 3,73 t/h CO₂ zurückgeführt. Die aus dem SMR Reaktor 6b gelangenden Gase werden einer Prozessgasaufbereitung 28 zur Trocknung, CO₂-Abtrennung sowie CO-H₂ Trennung zugeführt. Hierbei werden technologisch etablierte Verfahren eingesetzt. Aus der Prozessgasaufbereitung 28 werden 11,36 t/h Kohlenmonoxid (CO) 7 sowie 2,31 t/h Wasserstoff, 11,3 t/h Wasser, 3,73 t/h CO₂ sowie ein Restgas von 0,92 t/h enthaltend H₂, CO, nicht umgesetztes Methan abgetrennt. Von dem der Bereitstellung von Wasserstoff 3 zugeführten Wasserstoff werden 2,15 t/h Wasserstoff 3a der Hydrierung 20 der Dinitrotoluole 19a zugeführt. Die 0,16 t/h Wasserstoff 3a aus dem SMR Reaktor 6b bzw. dessen Prozessgasaufbereitung 28 werden der Ammoniaksynthese 21 zugeführt. 11,36 t/h CO 7 werden der Phosgensynthese 1 zugeführt. Hierbei wird das CO 7 mit Chlor 8b zu 35,56 t/h Phosgen 1a (COCI2) umgesetzt. In der Hydrierung 20 werden 32,68 t/h Dinitrotoluol 19a mit 2,15 t/h Wasserstoff 3a unter Abspaltung von 12,93 t/h Wasser zu 21,91 t/h Diaminotoluol 17 umgesetzt. Das Diaminotoluol 17 wird mit 35,56 t/h Phosgen 1a unter Abspaltung von 26,22 t/h Chlorwasserstoff zu 31,24 t/h Toluol-diisocyanat 18 umgesetzt. Aus 16,52 t/h Toluol 19c werden mit Salpetersäure 22a, gerechnet als 100% HNO₃ mit 22,62 t/h HNO₃ zu 32,68 t/h Dinitrotoluol 19a umgesetzt.

Für eine effektivere Wasserstofferzeugung wird ein SMR Reaktor (6a) nur für die H₂ Produktion eingesetzt, in dem das erzeugte CO mittels water-gas-shift Reaktion mit Wasser zu H₂ und CO₂ umgesetzt wird. Diesem Prozess werden 2,04 t/h Methan aus Erdgas und 7,78 t/h Wasser zugeführt. Es werden 0,92 t/h Wasserstoff sowie 5,28 t/h CO₂ und ein Restgas von 0,26 t/h bestehend aus CO, H₂ und Nebenprodukten wie Methan erhalten. Der Wasserstoff wird zusammen mit überschüssigem Wasserstoff 3a aus der SMR 6 der CO Herstellung von 0,16t/h in einer Ammoniaksynthese 21 mit 5,03 t/h N₂ 21a aus einer Luftzerlegung 23 zu 6,10 t/h Ammoniak 21b nach dem Haber-Bosch-Verfahren umgesetzt.

Anschließend wird der Ammoniak 21b durch Verbrennung, d.h. durch Umsatz mit 22,98 t/h Sauerstoff 5f für das sauerstoffhaltige Gas 5e zu Salpetersäure umgesetzt, die durch die Herstellung von Salpetersäure 22a der Herstellung 19 von Di-Nitrotoluol zugeführt wird.

In diesem Prozess werden 5,28 t/h CO₂ aus der Wasserstofferzeugung, der für die NH₃ Erzeugung eingesetzt wird, direkt frei gesetzt. Der Kohlenstoff aus der Isocyanatgruppe in dem organischen Isocyanat 18 stammt dabei aus einer fossilen Rohstoffquelle, dem Erdgas, und ist damit nicht nachhaltig. Der Kohlenstoff der Isocyanatgruppe beträgt 22% des Kohlenstoffes des TDIs.

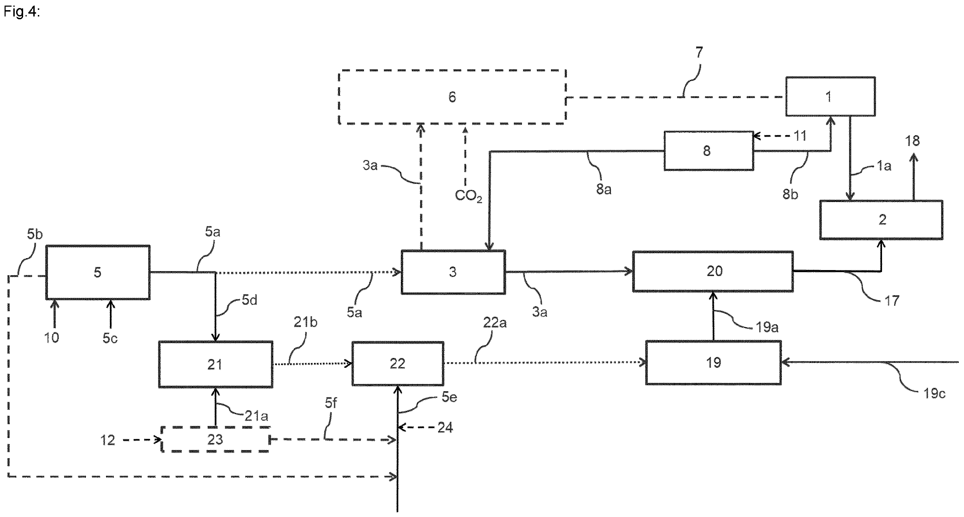

### Beispiel 2 Erfindungsgemäßes Verfahren - Gesamtprozess befindet sich an einem Standort (Fig. 4)

Die Verfahrensschritte des Beispiels 4 werden allesamt an einem Standort ausgeführt. Die gepunkteten Pfeile für 5a, 21b und 22a aus Fig. 4 bedeuten gemäß diesem Beispiel eine für die Bereitstellung der jeweiligen Stoffe genutzte Fluidverbindung und könnten auch mit einer durchgezogenen Linie symbolisiert werden.

Erfindungsgemäße Herstellung von emissionsarmen Toluol-diisocyanat (TDI), CO Herstellung bei dem in einem Reformerprozess 6 (rWGS reverse water gas shift reaction) in den CO₂ und H₂ als Rohstoffe eingesetzt werden. Optional kann die rWGS mit regenerativer elektrische Energie oder mit Bio-Erdgas beheizt werden. Der benötigte Wasserstoff und das Koppelprodukt Sauerstoff wird durch eine Wasserelektrolyse 5 erzeugt. Hierbei der erzeugte Wasserstoff bereitgestellt 3. Der Wasserstoff 5a aus der Wasserelektrolyse 5 wird dabei teilweise als Wasserstoff 5d mit Stickstoff 21a aus der Luftzerlegung 23 nach dem bekannten Haber-Bosch-Verfahren in der Ammoniaksynthese 21 zu Ammoniak 21b umgesetzt. Der Ammoniak 21b wird mit Luft 24 oder O₂, ggf. Sauerstoff 5f aus der Luftzerlegung 23 oder auch dem Sauerstoff 5b aus der Wasserelektrolyse 5 in der Herstellung von Salpetersäure 22 zu Salpetersäure umgesetzt. Der übrige Wasserstoff 5a aus der Wasserelektrolyse 5 wird für den RWGS-Reformer 6 und die Hydrierung 20 der Nitroaromaten 19a bereitgestellt 3. Zum Betrieb der Wasserelektrolyse 5 als auch der Luftzerlegung 23 wird regenerative Energie (10, 12) eingesetzt.

In einem rWGS Reaktionsraum 6, der bei einer Temperatur von 802°C betrieben wird, werden 17,84 t/h CO₂ sowie 0,81 t/h H₂ eingebracht. Aus der rWGS Reaktion 6 wird das erhaltene Produktgasgemisch bestehend aus CO 7, H₂O, nicht umgesetztem CO₂ sowie nicht umgesetzter H₂ sowie Nebenprodukten, hauptsächlich geringe Mengen an Methan, entnommen und einer Prozessgasaufbereitung (nicht gezeichnet 28 zugeführt. Hierbei wird CO₂ sowie Wasser abgetrennt und CO von H₂ getrennt sowie Restgase abgetrennt.

Aus der Wasserelektrolyse 5 werden insgesamt 4,04 t/h Wasserstoff 5a und 32,32 t/h O₂ 5b entnommen, hierbei wird der Wasserelektrolyse 5 37 t/h Wasser 5c zugeführt. Wenn sich die Wasserelektrolyse 5 am Standort der rWGS 6, Isocyanat-Produktion 2, Hydrierung 20 und Nitroverbindungsherstellung 19, NH₃ Synthese 22 und HNO₃ Herstellung 21 befindet, kann Wasser aus der Hydrierung 22, der Herstellung der Nitroverbindungen 21 sowie der Wasserelektrolyse 5 jeweils abgetrennt und der Wasserelektrolyse 5 zugeführt werden. Hierdurch wird die Ressource Wasser geschützt, eine weiterer Schritt, der die Nachhaltigkeit des erfindungsgemäßen Verfahrens verbessert.

Der rWGS 6 entnommene Gasstrom wird in der Prozessgasaufbereitung getrennt. Hierbei wird aus der H₂-CO Trennung 11,36 t/h CO einer Phosgensynthese 1 zugeführt. Hierfür wird das CO 7 mit Chlor 8b zu 35,56 t/h Phosgen 1a (COCI2) umgesetzt. In der Hydrierung 20 werden 32,68 t/h Dinitrotoluol 19a und 2,15 t/h Wasserstoff 3a aus der Wasserelektrolyse (5) unter Abspaltung von 12,93 t/h Wasser zu 21,91 t/h Diaminotoluol umgesetzt. Das Diaminotoluol wird mit 35,56 t/h Phosgen unter Abspaltung von 26,22 t/h Chlorwasserstoff zu 31,24 t/h Toluol-diisocyanat umgesetzt. Aus 16,52 t/h Toluol werden mit Salpetersäure, gerechnet als 100% HNO₃ mit 22,62 t/h HNO₃ zu 32,68 t/h Dinitrotoluol umgesetzt.

Für die Erzeugung von H₂ wird die Wasserelektrolyse (5) eingesetzt. In der Wasserelektrolyse werden 37 t/h Wasser zu 4,04 t/h Wasserstoff und 32,32 t/h Sauerstoff umgesetzt. Der Wasserstoff wird abei in die rWGS (6) mit 0,81 t/h zugeführt, der AmmoniakSynthese (21) mit 1,08 t/h sowie der Hydrierung der Nitroverbindungen (20) mit 2,15 t/h zugeführt.

Der Stickstoff 21a zur Herstellung von Ammoniak 21b in der NH₃-Synthese 21 wird aus der Luftzerlegung 23 bezogen, diese wird mit regenerativer Energie 12 betrieben. Aus der Luftzerlegung 23 werden 5,03 t/h Stickstoff 21a der Ammoniaksynthese 21 zugeführt und hier mit 1,08 t/h H₂ 5d aus der Wasserelektrolyse 5 zu 6,1 t/h Ammoniak 21b umgesetzt. Der Ammoniak wird über eine Fluidverbindung 21b von der Ammoniaksynthese 21 zur Herstellung von Salpetersäure 22 bereitgestellt.

Bei der Salpetersäureherstellung 22 kann das Koppelprodukt Sauerstoff 5b aus der Wasserelektrolyse 5 eingesetzt und damit die Umsetzung von 6,1 t/h Ammoniak 21b zu 22,6 t/h Salpetersäure, als 100%ige Säure gerechnet, erfolgen. Die Salpetersäure wird der Herstellung der Nitroverbindungen 19 über eine Fluidverbindung 22a zugeführt.

In diesem Prozess werden keine direkten CO₂ Emissionen aus der Wasserstofferzeugung frei gesetzt. Der Kohlenstoff aus der Isocyanatgruppe stammt dabei aus CO₂ und ist damit nachhaltig eingeführt. Es können damit die direkten Emissionen von 6,29 t/h CO₂ vermieden werden und 22% des Kohlenstoffes des TDI nachhaltig erzeugt werden.

## Patentansprüche

1. Verfahren zur Herstellung von organischer Aminoverbindung für die Synthese organischer Isocyanatverbindungen, enthaltend mindestens die Schritte
i) Bereitstellung mindestens einer organischen Nitroverbindung (19a) als unmittelbares Verfahrensprodukt eines Verfahrens zur Synthese (19) mindestens einer organischen Nitroverbindung (19a), das mindestens folgende Schritte umfasst:
i-1) Bereitstellung von Salpetersäure (22a) als unmittelbares Verfahrensprodukt eines Verfahrens zur Salpetersäureherstellung, das mindestens folgende Schritte umfasst:
a) Bereitstellung von Wasserstoffgas (5d) und Sauerstoffgas (5b) durch Ausführung einer Wasserelektrolyse (5) in einer Elektrolysevorrichtung unter Einsatz von aus regenerativer Energie hergestellter elektrischer Energie (10) und Wasser (5c);
b) Bereitstellung von Ammoniak (21b) als unmittelbares Verfahrensprodukt einer Umsetzung von zumindest Stickstoff (21a) und Wasserstoffgas (5d) aus besagter Wasserelektrolyse (5) in einer Ammoniaksynthese (21);
c) Herstellung von Salpetersäure durch Umsetzung von zuvor bereitgestelltem Ammoniak (21b) in einer Salpetersäure-Herstellung (22) zumindest mit Sauerstoff-haltigem Gas (5e);
i-2) Synthese (19) mindestens einer organischen Nitroverbindung (19a), bevorzugt mindestens einer aromatischen organischen Nitroverbindung (19a), zumindest durch Nitrierung mindestens einer organischen Verbindung (19b, 19c), bevorzugt mindestens einer aromatischen organischen Verbindung (19b, 19c), mit mindestens der besagten bereitgestellten Salpetersäure (22a);
ii) Bereitstellung (3) von Wasserstoffgas (3a), bevorzugt durch Ausführung einer Wasserelektrolyse in einer Elektrolysevorrichtung (3b) unter Einsatz von aus regenerativer Energie (9) hergestellter elektrischer Energie;
iii) Einbringung der mindestens einen bereitgestellten organischen Nitroverbindung (19a) und des bereitgestellten Wasserstoffgases in eine Hydrierung (20) und anschließende Hydrierung (20) durch Reduktion von Nitrogruppen der besagten mindestens einen organischen Nitroverbindung (19a) über deren Umsetzung mit besagtem Wasserstoffgas (3a) unter Erhalt mindestens einer organischen Aminoverbindung (17), bevorzugt mindestens einer aromatischen organischen Aminoverbindung (17).

2. Verfahren gemäß Anspruch 1, **dadurch gekennzeichnet, dass** die organische Nitroverbindung (19a) als Verfahrensprodukt besagter Synthese (19) des Schritts i-2) in Form eines Stoffstromes direkt über eine Fluidverbindung aus der besagten Synthese (19) über eine gegebenenfalls notwendige Zwischenlagerung in mindestens einem Lagerbehälter bereitgestellt wird, wobei die besagte Synthese (19), der gegebenenfalls notwendige mindestens eine Lagerbehälter und die Hydrierung (20) gemäß Schritt iii) miteinander in Fluidverbindung stehen.

3. Verfahren gemäß Anspruch 1, **dadurch gekennzeichnet, dass** die organische Nitroverbindung (19a) des Schritts i-2) als Verfahrensprodukt der besagten Synthese (19) in einem beweglichen, abgeschlossenem Transportbehälter bereitgestellt wird, gegebenenfalls daraus zur Zwischenlagerung in Lagerbehälter überführt wird, wobei die besagte Synthese (19) und die Hydrierung (20) gemäß Schritt iii) nicht miteinander in Fluidverbindung stehen.

4. Verfahren gemäß einem der Ansprüche 1 bis 3, **dadurch gekennzeichnet, dass** in Schritt i-1) die Salpetersäure als Verfahrensprodukt besagter Salpetersäureherstellung (22) in Form eines Stoffstromes (22a) direkt über eine Fluidverbindung aus der besagten Salpetersäureherstellung (22) über eine gegebenenfalls notwendige Zwischenlagerung in mindestens einem Lagerbehälter bereitgestellt wird, wobei die besagte Salpetersäureherstellung (22), der gegebenenfalls notwendige mindestens eine Lagerbehälter und die Synthese (19) gemäß Schritt i-2) über eine Fluidverbindung miteinander verbunden sind.

5. Verfahren gemäß Anspruch 4, **dadurch gekennzeichnet, dass** in Schritt i-1) der Ammoniak (21b) als Verfahrensprodukt der besagten Herstellung von Ammoniak (21) in einem beweglichen, abgeschlossenem Transportbehälter bereitgestellt wird, gegebenenfalls daraus zur Zwischenlagerung in Lagerbehälter überführt wird, wobei die besagte Herstellung von Ammoniak (21) und die Salpetersäureherstellung (22) nicht miteinander in Fluidverbindung stehen.

6. Verfahren gemäß Anspruch 1 bis 3, **dadurch gekennzeichnet, dass** in Schritt i-1) die Salpetersäure als Verfahrensprodukt der besagten Salpetersäureherstellung (22) zur Bereitstellung (22a) in einem beweglichen, abgeschlossenem Transportbehälter bereitgestellt wird, gegebenenfalls daraus zur Zwischenlagerung in Lagerbehälter überführt wird,
wobei die besagte Salpetersäureherstellung (22) und die Synthese (19) gemäß Schritt ii) nicht miteinander in Fluidverbindung stehen.

7. Verfahren nach einem der Ansprüche 1 bis 6, **dadurch gekennzeichnet, dass** der in Schritt ii) bereitgestellte Wasserstoff mindestens durch eine Elektrolyse von Wasser (3b) unter Einsatz von elektrischer Energie (9) hergestellt wird, wobei die Bereitstellung elektrischer Energie (9) als regenerative Energie wahlweise Windkraft, Sonnenenergie, Wasserkraft oder Mischungen daraus, eingesetzt wird.

8. Verfahren nach einem der vorangehenden Ansprüche, **dadurch gekennzeichnet, dass** die in Schritt iii) erhaltene organische Aminoverbindung (17) ausgewählt wird aus Toluylendiamin (TDA), Methylendi(phenylamin) (MDA) (wiederum bevorzugt ausgewählt aus Diphenylmethan-2,2'-diamin, Diphenylmethan-2,4'-diamin, Diphenylmethan-4,4'-diamin oder Mischungen daraus) oder Mischungen daraus.

9. Verfahren nach einem der vorangehenden Ansprüche, **dadurch gekennzeichnet, dass** für die Synthese organischen Isocyanats zusätzlich mindestens folgende Schritte ausgeführt werden:
iv) Synthese (1) von Phosgen (1a) aus zumindest Kohlenmonoxid und Chlorgas (8b);
v) Isocyanatherstellung (2) von organischem Isocyanat (18) durch Umsetzung zumindest des Phosgens (1a) mit der mindestens einen organischen Aminoverbindung (17), unter Freisetzung von Chlorwasserstoff.

10. Verfahren zur Synthese organischen Isocyanats (18), enthaltend mindestens die Schritte
Bereitstellung mindestens einer organischen Aminoverbindung (17) als unmittelbares Verfahrensprodukt eines Verfahrens nach einem der Ansprüche 1 bis 8;
Synthese (1) von Phosgen (1a) aus zumindest Kohlenmonoxid und Chlorgas (8b);
Isocyanatherstellung (2) von organischem Isocyanat (18) durch Umsetzung zumindest des Phosgens (1a) mit der mindestens einen organischen Aminoverbindung (17), unter Freisetzung von Chlorwasserstoff.

11. Verfahren nach einem der Ansprüche 9 oder 10, **dadurch gekennzeichnet, dass** das Kohlenmonoxid aus CO₂ hergestellt wird, zumindest durch mindestens ein Verfahren (6), ausgewählt aus
A) der elektrochemischen Reduktion von CO₂ unter Einsatz von aus regenerativer Energie hergestellter elektrischer Energie,
B) einem Reformerprozess, worin Methan und Wasserdampf unter Zusatz von mindestens CO₂, unter Zuführung von Wärmeenergie bei einer Temperatur von mindestens 500°C zu Kohlenmonoxid umgesetzt werden,
C) Umsetzung von Wasserstoff und CO₂ in einer reverse water-gas shift RWGS-Reaktionszone zu Kohlenmonoxid.

12. Verwendung von Salpetersäure, die ein unmittelbares Verfahrensprodukt eines Verfahrens zur Salpetersäureherstellung ist, das mindestens folgende Schritte umfasst:
a) Bereitstellung von Wasserstoffgas (5d) und Sauerstoffgas (5b) durch Ausführung einer Wasserelektrolyse (5) in einer Elektrolysevorrichtung unter Einsatz von aus regenerativer Energie hergestellter elektrischer Energie (10) und Wasser (5c);
b) Bereitstellung von Ammoniak (21b) durch Umsetzung von zumindest Stickstoff (21a) und Wasserstoffgas (5d) aus besagter Wasserelektrolyse (5) in einer Ammoniaksynthese (21);
c) Herstellung von Salpetersäure (22b) durch Umsetzung von zuvor hergestelltem Ammoniak (21b) in einer Salpetersäure-Herstellung (22) zumindest mit Sauerstoff-haltigem Gas (5e), bevorzugt mindestens ein Sauerstoff-haltiges Gas, ausgewählt aus Sauerstoff (5b) aus besagter Wasserelektrolyse (5), Luft (24) oder Sauerstoff (5f) aus einer Luftzerlegung (23);
zur Herstellung organischer Aminoverbindung für die Synthese von organischer Isocyanatverbindung.

13. Vorrichtung konfiguriert zur Herstellung organischer Aminoverbindungen, wobei die Vorrichtung mindestens eine Vorrichtung zur Bereitstellung von Wasserstoffgas, mindestens eine Vorrichtung zur Herstellung von organischen Nitroverbindungen, mindestens eine Vorrichtung zur Hydrierung von organischen Nitroverbindungen und mindestens eine Bereitstellung von Salpetersäure enthält, wobei
- die mindestens eine Vorrichtung zur Bereitstellung von Salpetersäure mindestens einen Auslass für Salpetersäure und mindestens einen Einlass für Salpetersäure und mindestens ein Volumen für Salpetersäure enthält;
- die mindestens eine Vorrichtung zur Herstellung von organischen Nitroverbindungen mindestens einen Reaktor, mindestens einen Einlass für organische Verbindungen, mindestens einen Einlass für Salpetersäure und mindestens einen Auslass für organische Nitroverbindungen enthält;
- die mindestens eine Vorrichtung zur Hydrierung von organischen Nitroverbindungen mindestens einen Reaktor, mindestens einen Einlass für organische Nitroverbindungen, mindestens einen Einlass für Wasserstoffgas und mindestens einen Auslass für organische Aminoverbindungen enthält;
**dadurch gekennzeichnet, dass**
i) der Lagerbehälter der Bereitstellung von Salpetersäure mit einer Salpetersäure befüllt ist, die ein unmittelbares Verfahrensprodukt eines Verfahrens zur Salpetersäureherstellung ist, das mindestens folgende Schritte umfasst:
a) Bereitstellung von Wasserstoffgas (5d) und Sauerstoffgas (5b) durch Ausführung einer Wasserelektrolyse (5) in einer Elektrolysevorrichtung unter Einsatz von aus regenerativer Energie hergestellter elektrischer Energie (10) und Wasser (5c);
b) Herstellung von Ammoniak (21b) durch Umsetzung von zumindest Stickstoff (21a) und Wasserstoffgas (5d) aus besagter Wasserelektrolyse (5) in einer Ammoniaksynthese (21);
c) Herstellung von Salpetersäure durch Umsetzung von zuvor hergestelltem Ammoniak (21b) in einer Salpetersäure-Herstellung (22) zumindest mit Sauerstoff-haltigem Gas (5e), bevorzugt mindestens ein Sauerstoff-haltiges Gas, ausgewählt aus Sauerstoff (5b) aus besagter Wasserelektrolyse (5), Luft (24) oder Sauerstoff (5f) aus einer Luftzerlegung (23);
ii) der Auslass für Salpetersäure der Vorrichtung zur Bereitstellung von Salpetersäure in Fluidverbindung mit dem Einlass für Salpetersäure der Vorrichtung zur Herstellung organischer Nitroverbindungen steht;
iii) der Auslass für organische Nitroverbindungen der Vorrichtung zur Herstellung organischer Nitroverbindungen in Fluidverbindung mit dem Einlass für organische Nitroverbindungen der Vorrichtung zur Hydrierung von organischen Nitroverbindungen steht;
iv) der Einlass für Wasserstoffgas der Vorrichtung zur Hydrierung von organischen Nitroverbindungen in Fluidverbindung mit dem Auslass für Wasserstoffgas der Vorrichtung zur Bereitstellung von Wasserstoffgas steht.

14. Vorrichtung nach Anspruch 13, **dadurch gekennzeichnet, dass** die Vorrichtung zur Bereitstellung von Wasserstoffgas (3) das Wasserstoffgas aus mindestens einer Elektrolyse zur Darstellung von Wasserstoffgas bezieht, ausgewählt aus einer Elektrolyse von Wasser, einer Elektrolyse von Alkalichlorid, einer Elektrolyse von Salzsäure oder aus mehreren der vorgenannten Elektrolysen.

15. Vorrichtung nach Anspruch 13 oder 14, **dadurch gekennzeichnet, dass** das Volumen für Salpetersäure der Vorrichtung zur Bereitstellung von Salpetersäure ein Volumen eines Lagerbehälters oder das Volumen einer Rohrleitung ist.

16. Vorrichtung nach Anspruch 13 oder 14, **dadurch gekennzeichnet, dass** das Volumen für Salpetersäure ein Volumen eines Lagerbehälters ist und der Einlass für Salpetersäure der Vorrichtung zur Bereitstellung von Salpetersäure in Fluidverbindung mit einer Entladevorrichtung für Transportbehälter steht.

17. Vorrichtung nach Anspruch 13 oder 14, **dadurch gekennzeichnet, dass** das Volumen für Salpetersäure eine Rohrleitung ist und der Einlass für Salpetersäure der Vorrichtung zur Bereitstellung von Salpetersäure in Fluidverbindung mit dem Auslass einer Vorrichtung für die Herstellung von Salpetersäure steht.

18. Vorrichtung nach einem der Ansprüche 13 bis 17, **dadurch gekennzeichnet, dass** der Auslass für organische Aminoverbindungen der Vorrichtung zur Hydrierung von organischen Nitroverbindungen in Fluidverbindung mit einem Einlass für organische Aminoverbindungen einer Vorrichtung zur Phosgenierung steht, wobei diese Vorrichtung zur Phosgenierung mindestens einen Einlass für Phosgen, mindestens einen Einlass für organische Aminoverbindungen und mindestens einen Auslass für organische Isocyanatverbindungen enthält.
